# EUROPÄISCHE PATENTANMELDUNG

(11) **EP 3 671 212 A2**
(43) Veröffentlichungstag der Anmeldung: **24.06.2020**
(21) Anmeldenummer: 19217068.6
(22) Anmeldetag: 17.12.2019
(51) Int. Cl.: G01N 33/564, G01N 33/543

(54) **VERFAHREN ZUR DIAGNOSE VON AUTOIMMUNGASTRITIS**

(30) Priorität: 18.12.2018 EP 18213659; 30.04.2019 EP 19171829
(71) Anmelder: Euroimmun Medizinische Labordiagnostika AG, 23560 Lübeck (DE)
(72) Erfinder: Dähnrich, Cornelia, 23627 Groß Grönau (DE); Komorowski, Lars, 23909 Ratzeburg (DE); Probst, Christian, 23909 Ratzeburg (DE)

(57) **Zusammenfassung**

Die vorliegende Erfindung betrifft ein Verfahren zur Diagnose von AIG umfassend den Schritt Nachweisen, ob in einer Probe, die Antikörper enthält, Autoantikörper gegen die beta-Untereinheit der gastritischen H⁺/K⁺-ATPase vorhanden oder nicht vorhanden sind, wobei ein diagnostisch nützlicher Träger mit einer Festphase eingesetzt wird, an der ein Mittel zum spezifischen Einfangen des Autoantikörpers immobilisiert ist, sowie einen diagnostisch nützlicher Träger mit einer Festphase, an der ein Mittel zum spezifischen Einfangen eines Autoantikörpers gegen die beta-Untereinheit der gastritischen H⁺/K⁺-ATPase immobilisiert ist.

## Beschreibung

Die vorliegende Erfindung betrifft ein Verfahren zur Diagnose von AIG umfassend den Schritt Nachweisen, ob in einer Probe, die Antikörper enthält, Autoantikörper gegen die beta-Untereinheit der gastritischen H⁺/K⁺-ATPase vorhanden oder nicht vorhanden sind, wobei ein diagnostisch nützlicher Träger mit einer Festphase eingesetzt wird, an der ein Mittel zum spezifischen Einfangen des Autoantikörpers immobilisiert ist, sowie einen diagnostisch nützlicher Träger mit einer Festphase, an der ein Mittel zum spezifischen Einfangen eines Autoantikörpers gegen die beta-Untereinheit der gastritischen H⁺/K⁺-ATPase immobilisiert ist.

Es ist anerkannt, dass zirkulierende Autoantikörper gegen diese H⁺/K⁺-ATPase aus den Parietalzellen, auch als APCA, Abkürzung für "anti-parietal cell antibodies" bezeichnet, diagnostische Marker für Autoimmungastritis (AIG) und perniziöse Anämie sind. AIG ist eine chronische Krankheit mit einer Prävalenz von ca. 2% in der Gesamtbevölkerung. In Patienten, die an anderen Autoimmunkrankheiten wie Typ I-Diabetes leiden, beträgt die Prävalenz bis zu 10%.

Die H⁺/K⁺-ATPase in den Parietalzellen der Lamina propria mucosae der Magenschleimhaut hat die Aufgabe, Protonen zu pumpen und damit ein saures Milieu im Magen aufrechtzuerhalten. Es handelt sich um ein Heterodimer aus einer alpha-Untereinheit (kodiert durch das ATP4A-Gen), welche das katalytische Zentrum enthält und den Ionen-Transport bewerkstelligt, und einer beta-Untereinheit (kodiert durch ATP4B), die die alpha-Untereinheit stabilisiert und für die enzymatische Aktivität notwendig ist.

Bei der AIG treten Verletzungen der Magenschleimhaut auf, die als Atrophische Gastritis bezeichnet werden (Atrophic body gastritis, ABG). Der Abbau der Schleimhaut führt dabei zu einer verringerten oder fehlenden Sekretion von Magensäure. Sie kann auch durch Infektionen durch *Helicobacter pylori* bedingt sein.

Neben anderen möglichen Ursachen kann eine Autoimmun-Gastritis eine Perniziöse Anämie (PA) bedingen, eine Anämie, die auf einem Mangel an Vitamin B12 beruht. Sie wird durch einen Mangel an intrinsischem Faktor bedingt, einem Protein, das das Vitamin bindet und stabilisiert und von den Parietalzellen gebildet wird, gegen die APCAs gerichtet sind.

Für die richtige Behandlung einer AIG bzw. PAist eine Differenzierung zwischen einer durch Autoimmunität bedingten, einer bakteriell oder durch Medikamente verursachten Gastritis unentbehrlich. Bei falscher oder ausbleibender Behandlung kann es zu einem Magengeschwür oder einem Magendurchbruch kommen, zudem steigt das Risiko eines Magenkarzinoms.

Es besteht daher Bedarf an Hochdurchsatz-tauglichen diagnostischen Tests, mit denen diagnostisch nützliche Antikörper in leicht verfügbarem Probenmaterial von Patienten mit möglichst hoher diagnostischer Verlässlichkeit nachgewiesen werden können, insbesondere mit möglichst hoher Sensitivität und/oder Spezifität, besonders gegenüber Tests, die auf dem Nachweis von einem Autoantikörper gegen die alpha-Untereinheit oder das Heterodimer umfassend alpha- und beta-Untereinheit der gastritischen H⁺/K⁺-ATPase basieren.

Es sei darauf hingewiesen, dass das Testergebnis allein keine Diagnose ermöglicht, nicht zuletzt, weil Proben von Patienten mit zahlreichen anderen Krankheiten wie Pankreatitis oder Diabetis mellitus den Autoantikörper aufweisen können. Das Testergebnis kann vom behandelnden Arzt zusammen mit weiteren Symptomen wie Vitamin B12-Mangel, hyperchromer Anämie, dem Infiltrieren der Magenschleimhaut mit Lymphozyten interpretiert werden und unterstützt damit die Diagnose, ohne selbst für eine endgültige Diagnose eine ausreichende Entscheidungsgrundlage zu stellen.

Im Stand der Technik sind dazu ELISA- und Immunfluoreszenz-Tests offenbart, mit denen Autoantikörper gegen das Heterodimer mit der ATP4A- und der ATP4B-Untereinheit nachgewiesen werden können.

Die EUROIMMUN Medizinische Labordiagnostika AG verkauft seit 2011 einen ELISA basierend auf aufgereinigter nativer Säugetier-ATP4 mit beiden Untereinheiten. In der Testbeschreibung wird die Spezifität ausführlich addressiert und darauf hingewiesen, dass man diese über die Wahl des Cut-Off-Wertes optimieren kann (Katalog Nr. EA 1361-9601 G).

Die WO2011/150086 offenbart den Nachweis von Autoantikörpern gegen die ATP4A-Untereinheit.

Wenzlau *et al.* offenbaren den Nachweis von Autoantikörpern gegen ATP4A zur Diagnose von Autoimmun-Gastritis in Patienten, die unter Typ I-Diabetes leiden. Es wurde ein Radiobindungs-Test eingesetzt (Wenzlau, J.M., Gardner, T.J., Frisch, L.M., Davidson, H.W. and Hutton, J.C. (2011) Development of a novel autoantibody assay for autoimmune gastritis in T1D individuals, Diabetes Metab Res Rev., 27 (8): 887-90).

Rusak *et al.* offenbaren, dass die ATP4A-Untereinheit der gastritischen H⁺/K⁺-ATPase das Hauptantigen für APCA ist (Rusak, E., Chobot, A., Krzywicka, A. and Wenzlau, J. (2016) Anti-parietal cell antibodies - diagnostic significance, Advances in Medical Sciences, 61:175-179). Sie geben an, dass mit Radioimmunassays bessere Ergebnisse als mit ELISA oder IFT erzielt werden können.

Scharf *et al.* offenbaren im Zusammenhang mit neurologischen Autoimmunkrankheiten, dass Autoantikörper gegen das ATPase-Heterodimer unter Verwendung eines Immunfluoreszenz-Tests nachgewiesen werden konnten, wenn Zellen eingesetzt wurden, die entweder ATP4A oder ATP4B exprimierten (Scharf, M., Miske, R., Heidenreich, F., Giess, R., Landwehr, P., Blöcker, I., Begemann, N., Denno, Y., Tiede, S., Dähnrich, C., Schlumberger, W., Unger, M., Teegen, B., Stöcker, W., Probst, C., Komorowski, L. (2015) Neuronal Na+/K+ ATPase is an autoantibody target in paraneoplastic neurologic syndrome, Neurology, 84:1-7). In einem Serum von einem Patienten mit neurologischen Symptomen wurden Antikörper gegen ATP4B, aber nicht gegen ATP4A nachgewiesen. Es wird nicht offenbart, welche Art von Antigenen genau eingesetzt wurde und wie das Verfahren durchgeführt wurde.

Lahner *et al.* offenbaren die Detektion von Autoantikörpern gegen ATP4A und ATP4B unter Verwendung eines Lumineszenz-Immunpräzipitations-System (LIPS) aus Proben von Patienten, die unter einer atrophischen Gastritis litten. Es wird herausgestellt, dass Autoantikörper gegen ATP4A und ATP4B beinahe mit der gleichen, hohen Sensitivität und Spezifität nachgewiesen werden können (Lahner, E., Brigatti, C., Marzinotto, I., Carabotti, M., Scalese, G., Davidson, H.W., Wenzlau, J.M., Bosi, E., Piemonti, L., Annibale, B. and Lampasona, V. (2017) Luminescent Immunoprecipitation System (LIPS) for Detection of Autoantibodies Against ATP4A and ATP4B Subunits of Gastric Proton Pump H+/K+-ATPase in Atrophic Body Gastritis Patients, Clinical and Translational Gastroenterology, 8(1):e215). Burbelo *et al.* untersuchten das Auftreten von Antikörpern gegen ATP4B bei Patienten, die unter Typ I-Diabetes litten (Burbelo, P.D., Lebovitz, E.E., Bren, K.E., Bayat, A., Paviol, S., Wenzlau, J.M., Barriga, K.J., Rewers, M., Harlan, D.M. and ladarola, M.J. (2012) Extrapancreatic Autoantibody Profiles in Type I Diabetes, PLOS ONE, 7(9):e45216).

Kontani *et al.* identifizierten unter Verwendung von Proben von Mäusen die alpha-Untereinheit als antigenes Hauptprotein in AIG (Kontani, K., Taguchi, D., und Takahashi, T. (1992) Involvement of the H+/+-ATPase alpha subunit as a major antigenic protein in autoimmune gastritis induced by neonatal thymectomy in mice, Clin. Exp. Immunol. 89, 63-67).

Ma *et al.* kommen unter Verwendung von unaufgereinigter beta-Untereinheit in Form eines Zellextraktes zu dem Schluss, dass sowohl die alpha- als auch die beta-Untereinheit an Autoantikörper von Patienten mit AIG binden. Ein direkter Vergleich der menschlichen alpha- und beta-Untereinheiten wurde nicht vorgenommen (Ma, J. Y., Borch, K und Mardh, S. (1994) Human Gastric H,K-Adenosine Triphosphatase beta-Subunit is a Major Autoantigen in Atrophic Corpus Gastritis, Scand J. Gastroenterol 29, 790-794).

Sowohl Lahner *et al.* als auch Burbelo *et al.* geben an, dass nur ein Test mit besonders hoher Sensitivität und Spezifität wie LIPS oder eine Radiobindungs-Test für eine zuverlässig Diagnostik geeignet ist, im Gegensatz zu Testsystemen mit Festphase, mit denen viele diagnostisch nützliche Epitope nicht nachgewiesen werden können. Es werden keine Gründe angegeben, warum nicht die ATPA4-Untereinheit ausgewählt wurde.

Die der Erfindung zu Grunde liegende Aufgabe wird durch den Gegenstand der beigefügten unabhängigen und abhängigen Ansprüche gelöst.

In einem erstem Aspekt wird das der Erfindung zu Grunde liegende Problem gelöst durch ein Verfahren zur Diagnose von AIG, optional zur Diagnose von AIG und/oder PA, umfassend den Schritt Nachweisen, ob in einer Probe, die Antikörper enthält, ein Autoantikörper oder Autoantikörper gegen die beta-Untereinheit der gastritischen H⁺/K⁺-ATPase vorhanden oder nicht vorhanden ist oder sind, wobei ein diagnostisch nützlicher Träger mit einer Festphase eingesetzt wird, an der ein Mittel zum spezifischen Einfangen des Autoantikörpers immobilisiert ist.

In einer bevorzugten Ausführungsform ist der Träger aus der Gruppe ausgewählt, die eine Mikrotiterplatte, einen Bead, einen Blot; bevorzugt Western Blot, Lineblot oder Dot Blot; einen Immunfluoreszenztestträger mit fixierten Zellen, bevorzugt für eine mikroskopische Analyse, eine Lateral Flow-Vorrichtung, ein zellulosebasiertes Polymer, einen Biochip, einen Mikroarray, und ein Chromatographiesäulenmaterial umfasst.

In einer bevorzugten Ausführungsform handelt es sich bei dem diagnostisch nützlichen Träger um eine Mikrotiterplatte für ELISA.

In einer bevorzugten Ausführungsform ist die Probe aus der Gruppe ausgewählt, die Serum, Vollblut, Plasma, CSF umfasst.

In einer bevorzugten Ausführungsform umfasst das Verfahren Nachweisen, ob in einer Probe ein Autoantikörper oder Autoantikörper gegen den Intrinsic Factor vorhanden oder nicht vorhanden ist oder sind.

In einer bevorzugten Ausführungsform ist der Autoantikörper ein Antikörper der Klasse IgG.

In einem zweiten Aspekt wird das der Erfindung zu Grunde liegende Problem gelöst durch einen diagnostisch nützlichen Träger mit einer Festphase, an der ein Mittel zum spezifischen Einfangen eines Autoantikörpers gegen die beta-Untereinheit der gastritischen H⁺/K⁺-ATPase immobilisiert ist.

In einem dritten Aspekt wird das der Erfindung zu Grunde liegende Problem gelöst durch einen monoklonalen Antikörper oder isolierten Autoantikörper, der spezifisch gegen die beta-Untereinheit der gastritischen H⁺/K⁺-ATPase bindet, besonders bevorzugt in aufgereinigter Form, bevorzugt aber nicht gegen die alpha-Untereinheit.

In einem vierten Aspekt wird das der Erfindung zu Grunde liegende Problem gelöst durch ein Verfahren zum Kalibrieren oder zur Kontrolle der Qualität eines Medizinproduktes umfassend den Schritt Kontaktieren des erfindungsgemäßen Trägers mit einer Flüssigkeit umfassend den erfindungsgemäßen Antikörper, bevorzugt in bekannter Konzentration, gefolgt von dem Schritt Nachweisen, ob der Antikörper in der Flüssigkeit vorhanden oder nicht vorhanden sind, besonders bevorzugt unter wenigstens semi-quantitativer Bestimmung des Antikörpers in der Flüssigkeit.

In einem fünften Aspekt wird das der Erfindung zu Grunde liegende Problem gelöst durch einen Kit umfassend den erfindungsgemäßen diagnostisch nützlichen Träger, wobei der Kit ein oder mehr als ein Reagenz, bevorzugt alle Reagenzien, aus der Gruppe enthält, die eine Waschlösung, eine Kalibratorlösung, einen Antikörper gegen die beta-Untereinheit der gastritischen H⁺/K⁺-ATPase, und ein Mittel zum Detektieren des Autoantikörpers, bevorzugt einen sekundären Antikörper, noch bevorzugter einen sekundären Antikörper gegen IgG-Antikörper, umfasst.

In einer bevorzugten Ausführungsform weist der sekundäre Antikörper eine nachweisbare Markierung auf. Die nachweisbare Markierung ist bevorzugt ausgewählt aus der Gruppe umfassend eine chemilumineszenzfähige, radioaktive oder ein nachweisbares Isotop umfassende Markierung, eine enzymatisch aktive Markierung, eine fluoreszenzfähige Markierung, eine durch NMR-Spektroskopie nachweisbare Verbindung, eine Spin Label-Markierung, bevorzugt eine chemilumineszenzfähige Markierung, am bevorzugtesten eine chemilumineszenzfähige Markierung, die bei Inkubation in einer geeigneten Chemilumineszenz-Triggerlösung ein Chemilumineszenz-Signal erzeugt.

In einem sechsten Aspekt wird das der Erfindung zu Grunde liegende Problem gelöst durch eine Verwendung eines Mittels zum Nachweisen, ob in einer Probe ein Autoantikörper oder Autoantikörper gegen die beta-Untereinheit der gastritischen H⁺/K⁺-ATPase vorhanden oder nicht vorhanden ist oder sind, oder des erfindungsgemäßen Trägers oder des erfindungsgemäßen Antikörpers zur hochspezifischen Diagnose von AIG und/oder PA.

In einem siebten Aspekt wird das der Erfindung zu Grunde liegende Problem gelöst durch die Verwendung eines Mittels zum Nachweisen, ob in einer Probe ein Autoantikörper oder Autoantikörper gegen die beta-Untereinheit der gastritischen H⁺/K⁺-ATPase vorhanden oder nicht vorhanden ist oder sind, oder eines Trägers umfassend das Mittel zur Herstellung eines Kits, einer Zusammensetzung für die hochspezifische Diagnose von AIG und/oder PA.

In einem achten Aspekt wird das der Erfindung zu Grunde liegende Problem gelöst durch die Verwendung eines Autoantikörpers gegen die beta-Untereinheit der gastritischen H⁺/K⁺-ATPase für die Diagnose, bevorzugt die hochspezifische Diagnose von AIG. Besonders bevorzugt dient dabei der Autoantikörper der beta-Untereinheit als alleiniger Biomarker, bevorzugt als alleiniger Autoimmun-Biomarker in einem Testdurchgang, noch bevorzugter als alleiniger von der gastritischen H⁺/K⁺-ATPase abgeleiteter Autoimmun-Biomarker. Das kann bedeuten, dass parallel kein weiterer Autoantikörper in der gleichen Probe nachgewiesen wird, besonders kein weiterer Autoantikörper gegen die gastritische H⁺/K⁺-ATPase, besonders die alpha-Untereinheit.

Die vorliegende Erfindung basiert auf der überraschenden Erkenntnis der Erfinder, dass das Nachweisen von Autoantikörpern mit optimierter diagnostischer Zuverlässigkeit, insbesondere Spezifität und Sensitivität, einen Beitrag zur Diagnose von AIG leisten kann, wenn nachgewiesen wird, ob ein Autoantikörper gegen die beta-Untereinheit der gastritischen H⁺/K⁺-ATPase, nicht etwa gegen die alpha-Untereinheit oder das Heterodimer vorhanden ist. Dies trifft auch dann zu, wenn ein diagnostisch nützlicher Träger mit einer Festphase eingesetzt wird, d.h. Assayformate, die im Stand der Technik als diagnostisch unzuverlässig dargestellt werden.

Die vorliegende Erfindung basiert weiterhin auf der überraschenden Erkenntnis der Erfinder, dass der Nachweis von Autoantikörpern gegen die extrazelluläre Domäne der beta-Untereinheit der gastritischen H⁺/K⁺-ATPase eine besonders spezifische Diagnose gestattet. Damit kann die Zahl falsch positiver Diagnosen oder Befunde auf hohem Niveau reduziert werden.

In einer bevorzugten Ausführungsform wird der Test unter Verwendung eines diagnostisch nützlichen Trägers mit einer Festphase durchgeführt, an der ein Mittel zum spezifischen Einfangen eines Autoantikörpers gegen die beta-Untereinheit der gastritischen H⁺/K⁺-ATPase immobilisiert ist. In einer bevorzugteren Ausführungsform handelt es sich bei dem Mittel um ein Polypeptid umfassend die beta-Untereinheit der gastritischen H⁺/K⁺-ATPase oder eine Variante davon. Jedoch sind auch davon abgeleitete oder das Rückgrat der beta-Untereinheit nachahmende Moleküle wie Peptidomimetika, Peptoide, beta-Peptide und Peptide mit unnatürlichen Aminosäuren verwendbar. Der Fachmann ist mit derartigen Molekülen und ihrem Design vertraut (Pelay Gimeno, M., Glas, A., Koch, O., Grossmann, T.N. (2015) Structure-based design of inhibitors of protein-protein interactions: Mimicking peptide binding epitopes. Angewandte Chemie International Edition. 54 (31): 8896-8927). Ebenso ist möglich, dass die beta-Untereinheit indirekt dadurch nachgewiesen wird, dass einerseits nachgewiesen wird, ob ein Autoantikörper oder Autoantikörper gegen das Heterodimer umfassend alpha- und beta-Untereinheit vorhanden oder nicht vorhanden ist oder sind, und andererseits nachgewiesen wird, ob ein Autoantikörper oder Autoantikörper gegen die alpha-Untereinheit vorhanden oder nicht vorhanden ist oder sind.

In einer bevorzugten Ausführungsform bezeichnet der Begriff "immobilisiert", wie hierin verwendet, dass das Mittel zum Nachweisen, ob ein Autoantikörper gegen die beta-Untereinheit der gastritischen H⁺/K⁺-ATPase vorhanden oder nicht vorhanden ist, an die Festphase des Trägers gebunden ist, bevorzugt über eine kovalente Bindung, elektrostatische Interaktion, oder über hydrophobe Interaktion, wobei das Mittel gegenüber der Umgebung derart exponiert bleibt, dass es für Autoantikörper in einer flüssigen Probe zugänglich ist. Verschiedene geeignete Träger, zum Beispiel Papier, Polystyrol, Metall, Silikon oder Glasoberflächen, mikrofluidische Kanäle, Membranen, Beads wie magnetische Beads, Chromatographie-Säulenmedien, Biochips, Polyacrylamid Gele und dergleichen sind in der Literatur beschrieben, zum Beispiel in Kim, D., and Herr, A. E. (2013), Protein immobilization techniques for microfluidic assays, Biomicrofluidics 7(4), 041501. Auf diese Weise kann das immobilisierte Mittel zusammen mit dem unlöslichen Träger von einer wässrigen Lösung auf einfache Weise getrennt werden, zum Beispiel durch Filtrierung, Zentrifugieren oder Dekantieren. Ein immobilisiertes Mittel kann reversibel oder irreversibel immobilisiert sein. Zum Beispiel ist die Immobilisierung reversibel, wenn das Mittel mit dem Träger über eine ionische Interaktion interagiert, die durch Hinzufügen einer hohen Konzentration eines Salzes maskiert werden kann, oder wenn das Mittel über eine spaltbare kovalente Bindung wie eine Disulfidbrücke gebunden ist, die durch Hinzufügen von Thiol enthaltenen Reagenzien gespalten werden kann. Im Gegensatz dazu ist die Immobilisierung irreversibel, wenn das Mittel an dem Träger über eine kovalente Bindung befestigt ist, die nicht in wässriger Lösung gespalten werden kann, zum Beispiel eine Bindung, die durch Reaktion einer Epoxidgruppe und einer Aminogruppe gebildet wird, wie sie häufig verwendet wird, um Lysinseitenketten an Affinitätssäulen zu binden. Das Mittel, bevorzugt ein Polypeptid, kann indirekt immobilisiert werden, zum Beispiel durch Immobilisieren eines Antikörpers oder einer anderen Einheit mit Affinität an das Mittel, gefolgt von der Bildung eines Komplexes mit der Wirkung, dass der Mittel-Antikörper-Komplex immobilisiert ist. Es kann auch direkt immobilisiert sein, d.h. zur Beschichtung des Trägers in direktem Kontakt. Verschiedene Möglichkeiten zum Immobilisieren sind in der Literatur beschrieben, zum Beispiel in Kim, D., and Herr, A. E. (2013), Protein immobilizsation techniques for microfluidic assays, Biomicrofluidics 7(4), 041501.

Bei der beta-Untereinheit der gastritischen H⁺/K⁺-ATPase handelt es sich bevorzugt um die mit dem UniProtKB-Sequenzeintrag P51164 oder in SEQ ID NO7 dargestellte Aminosäuresequenz, bevorzugter um die durch SEQ ID NO3 dargestellte Aminosäuresequenz, wobei die letztere die extrazelluläre Domäne darstellt, die die Transmembrandomäne nicht aufweist, noch bevorzugter um das in SEQ ID NO5 dargestellte Fragment. Erfindungsgemäß können Varianten davon eingesetzt werden. In dieser Anmeldung stehen sämtliche zitierte Datenbank-Codes für die Uniprot-Datenbank oder sonstige Datenbanken, genauer für deren Version am Einreichungstag dieser Anmeldung oder ihrer frühesten Prioritätsanmeldung. Besonders bevorzugt ist, dass SEQ ID NO3 oder eine Variante davon in Abwesenheit von wenigstens einem Epitop aus der Transmembrandomäne der beta-Untereinheit mit der Sequenz SEQ ID NO4 und/oder wenigstens irgendeinem Epitop der alpha-Untereinheit verwendet wird, das gegenüber einem Autoantikörper aus dem Serum eines AIG-Patienten reaktiv ist, bevorzugt irgendeinem Epitop der alpha-Untereinheit.

Bei der alpha-Untereinheit der gastritischen H⁺/K⁺-ATPase handelt es sich bevorzugt um die durch UniProtKB-Zugriffsnummer P20648 oder in SEQ ID NO9 dargestellte Aminosäuresequenz. Bei dem Intrinsic factor handelt es sich bevorzugt um die durch den NCBI-Genbankeintrag NP_005133.2 oder in SEQ ID NO10 dargestellte Aminosäuresequenz.

In einer bevorzugten Ausführungsform wird unter "Nachweisen, ob in einer Probe ein Autoantikörper gegen die beta-Untereinheit der gastritischen H⁺/K⁺-ATPase vorhanden oder nicht vorhanden ist", verstanden, dass nachgewiesen wird, ob die Probe einen Autoantikörper enthält, der gegen die beta-Untereinheit, nicht jedoch gegen die alpha-Untereinheit des gleichen Proteins gerichtet ist. Bevorzugt wird auch ein Autoantikörper, der nur gegen den Komplex umfassend alpha- und beta-Untereinheit gerichtet ist, nicht nachgewiesen. Dabei ist besonders bevorzugt, dass ein erstes Signal erzeugt wird, das anzeigt, dass der Autoantikörper gegen die beta-Untereinheit gebunden hat und von einem Signal unterschieden werden kann, das dadurch entsteht, dass ein anderer Autoantikörper an ein anderes Autoantigen wie die alpha-Untereinheit oder an ein Autoantigen gebunden hat, das aus Aminosäuren der alpha- und der beta-Untereinheiten besteht. Es kann also unterschieden werden, ob ein für die beta-Untereinheit spezifischer Antikörper an die beta-Untereinheit bindet, oder ob ein anderes Ereignis stattgefunden hat, besonders die Bindung eines Autoantikörpers, der gegen die alpha-Untereinheit oder den Komplex aus alpha- und beta-Untereinheit, nicht aber die beta-Untereinheit allein bindet. In einer bevorzugten Ausführungsform wird erfindungsgemäß statt eines Mittels zum spezifischen Einfangen ein Mittel zum spezifischen Nachweis, ob in einer Probe ein Autoantikörper gegen die beta-Untereinheit der gastritischen H⁺/K⁺-ATPase vorhanden oder nicht vorhanden ist, eingesetzt, wobei es sich bevorzugt um ein Mittel handelt, dass den Nachweis gestattet, ob in einer Probe ein Autoantikörper gegen die beta-Untereinheit der gastritischen H⁺/K⁺-ATPase vorhanden oder nicht vorhanden ist. Das Mittel zum Nachweisen oder das Mittel zum spezifischen Einfangen kann ein Polypeptid oder ein Reagenz oder eine Zusammensetzung zum Nachweisen bzw. spezifischen Einfangen sein.

Der Nachweis des Autoantikörpers kann qualitativ oder quantitativ sein. In einer bevorzugten Ausführungsform wird unter dem Begriff "qualitativ" verstanden, dass lediglich nachgewiesen wird, ob der Autoantikörper anwesend ist oder nicht, nicht jedoch, in welcher Konzentration. Insbesondere wird nicht festgestellt, ob die Konzentration des Autoantikörpers einen gewissen Wert überschreitet, der weitere Information zur vorliegenden Erkrankung erbringt, beispielsweise zur Schwere der Krankheit, zur Entwicklung, zum Therapieerfolg oder zu bestimmten Symptomen. In einer bevorzugten Asuführungsform wird unter dem Begriff "quantitativ" verstanden, dass eine Aussage hinausgehend über diejenige getroffen wird, dass der Autoantikörper nachgewiesen werden kann. Insbesondere kann ein absoluter oder relativer Wert bestimmt werden oder wenigstens eine Zuordnung zu einem von mehreren definierten Konzentrationsbereichen.

Dies kann dadurch erreicht werden, dass die beta-Untereinheit oder eine Variante davon räumlich getrennt von anderen Antigenen, bevorzugt Autoantigenen oder gegenüber Autoantikörpern aus Proben von AIG-Patienten reaktiven Epitopen solcher Autoantigene eingesetzt wird, insbesondere der alpha-Untereinheit oder Varianten davon. Alternativ kann nachgewiesen werden, ob in der Probe ein Autoantikörper vorhanden ist, der gegen den Komplex umfassend alpha- und beta-Untereinheit der gastritischen H⁺/K⁺-ATPase bindet, und zusätzlich, ob in der Probe kein Antikörper vorhanden ist, der spezifisch gegen die alpha-Untereinheit bindet. Ist das nämlich der Fall, so kann geschlussfolgert werden, dass ein Autoantikörper vorhanden ist, der spezifisch gegen die beta-Untereinheit bindet, auch wenn weder die beta-Untereinheit noch eine Variante davon für den Test eingesetzt wird. Schließlich besteht auch die Möglichkeit, dass sämtliche in der Probe vorhandenen Antikörper der gesuchten Klasse, bevorzugt IgG, immobilisiert werden, gefolgt vom Kontaktieren mit der beta-Untereinheit der gastritischen H⁺/K⁺-ATPase, die entweder selbst markiert ist oder über einen markierten Liganden, bevorzugt einen markierten Antikörper, nachgewiesen werden kann.

Für die Einschätzung, ob ein Patient ein erhöhtes Risiko hat, unter AIG und/oder PA zu leiden oder zu erkranken, wird bevorzugt ausschließlich durch die Anwesenheit oder Abwesenheit von Autoantikörpern gegen die beta-Untereinheit angezeigt, ob dies der Fall ist. Hingegen findet die Detektion von Autoantikörpern gegen die alpha-Untereinheit keine Beachtung, weil sie kein derartig erhöhtes Risiko anzeigt.

In einer bevorzugten Ausführungsform wird unter dem "Mittel zum spezifischen Einfangen eines Autoantikörpers gegen die beta-Untereinheit der gastritischen H⁺/K⁺-ATPase" ein Mittel verstanden, mit dem ein Autoantikörper gegen die beta-Untereinheit der gastritischen H⁺/K⁺-ATPase eingefangen werden kann, andere Antikörper, bevorzugt Autoantikörper, besonders gegen die alpha-Untereinheit jedoch weniger effizient, am bevorzugtesten nicht oder in einem nicht detektierbaren Ausmaß, so dass sie bei einem erfindungsgemäßen Nachweisverfahren beim Kontaktieren des Mittels oder Trägers umfassend das Mittel mit der Probe uneingefangen in der Probe verbleiben. Bevorzugt bindet das Mittel zum spezifischen Einfangen eines Autoantikörpers gegen die beta-Untereinheit der gastritischen H⁺/K⁺-ATPase spezifisch an einen Autoantikörper gegen die beta-Untereinheit aus einer Probe von einem AIG-Patienten. In einer bevorzugten Ausführungsform werden unter allen Autoantikörpern gegen die H⁺/K⁺-ATPase nur diejenigen verwendet bzw. als Marker verwendet, um AIG zu diagnostizieren, die gegen die beta-Untereinheit der gastritischen H⁺/K⁺-ATPase binden, insbesondere bevorzugt nicht diejenigen, die gegen die alpha-Untereinheit binden. Dieser Autoantikörper bzw. diese Autoantikörper dienen als alleiniger Autoantikörper-Marker zur erfindungsgemäßen Diagnose. Bevorzugt sind von der alpha-Untereinheit abgeleitete Epitope, an die Autoantikörper gegen die alpha-Untereinheit binden, nicht aber Autoantikörper, die gegen die beta-Untereinheit binden, abwesend.

In einer besonders bevorzugten Ausführungsform bedeutet der Begriff "spezifisch binden", wie hierin verwendet, bei einem Antikörper, dass er gegen das entsprechende Antigen in einer Bindungsreaktion bindet, die durch eine Dissoziationskonstante gekennzeichnet ist, die 1 x 10⁻⁵ M, bevorzugter 1 x 10⁻⁷ M, bevorzugter 1 x 10⁻⁸ M, bevorzugter 1 x 10⁻⁹ M, bevorzugter 1 x 10⁻¹⁰ M, bevorzugter 1 x 10⁻¹¹ M, bevorzugter 1 x 10⁻¹² M beträgt oder eine stärkere Bindungsreaktion. Bevorzugt wird die Dissoziationskonstante dabei durch Surface Plasmon Resonanz unter Verwendung eines Biacore-Gerätes bei 25 °C und in PBS-Puffer bei pH 7 gemessen und unter Verwendung der vom Hersteller angebotenen Software berechnet.

In einer bevorzugten Ausführungsform wird unter dem Begriff "Diagnose", wie hierin verwendet, eine Vorgehensweise verstanden, bei der Information gewonnen wird, die die Einschätzung unterstützt oder überhaupt erst die Einschätzung gestattet, ob ein Subjekt unter einer Krankheit leidet oder mit höherer Wahrscheinlichkeit als eine durchschnittliche Person unter einer Krankheit leidet oder in der Vergangenheit litt oder in der Zukunft leiden wird, ebenso, ob eine Krankheit fortschreitet oder wie sie sich in der Zukunft entwickeln wird oder um einzuschätzen, ob eine bestimmte Behandlung anschlagen wird. Von dem Subject ist vor der Diagnose bevorzugt nicht bekannt, ob es unter der Krankheit leidet.

Bevorzugt ist es ausreichend für die Diagnose oder zum Unterstützen der Diagnose, lediglich nachzuweisen, ob der Antikörper vorhanden ist, wobei bestimmt werden kann, ob nachweisbare Konzentrationen des Antikörpers in der Probe vorhanden sind. In einer bevorzugten Ausführungsform wird bestimmt, ob die relative Konzentration des Antikörpers bei einem zu diagnostizierenden Patienten höher ist als bei einem durchschnittlichen Gesunden. Es kann bestimmt werden, ob die Konzentration um den Faktor 1,1, bevorzugter 1,2, 1,5, 1, 2, 5, 10, 20, 25, 50, 100, 200, 500, 1000, 10000 or 100000 höher ist als in einer Probe von einem durchschnittlichen Gesunden. Die höhere Konzentration stützt die Diagnose, dass der Patient, von dem die Probe stammt, an AIG leidet bzw. zeigt an, dass der Patient mit erhöhter Wahrscheinlichkeit an AIG leidet.

Das erfindungsgemäße Verfahren oder die erfindungsgemäße Verwendung erfolgt bevorzugt *in vitro.*

Der Fachmann versteht, dass ein solcher Nachweis in der Regel nicht allein eine umfassende Diagnose gestattet, sondern dass weitere Aspekte berücksichtigt werden müssen, beispielsweise weitere in einer Probe zu bestimmende Parameter, die medizinische Vorgeschichte, klinische Symptome, die Anamnese oder die Ergebnisse bildgebender Verfahren. Er versteht weiter, dass der Wert des erfindungsgemäßen Verfahrens darin bestehen kann, dass eine indirekte Diagnose oder Differentialdiagnose erfolgen kann, bei der der Ausschluss einer Krankheit anzeigt, dass der Patient unter einer anderen Krankheit mit ähnlichen Symptomen leidet. In einer bevorzugten Ausführungsform dienen erfindungsgemäße Produkte wie Träger oder Kits oder erfindungsgemäße Verfahren zur Differenzierung zwischen einer durch Autoimmunität bedingten, einer bakteriell oder durch Medikamente verursachte Gastritis oder zur Diagnose einer Krankheit aus der Gruppe umfassend AIG, Perniziöse Anämie, Atrophische Gastritis oder Vitamin B12-Mangel.

Die zu untersuchende Probe ist für den Fall, dass Antikörper nachgewiesen werden, eine Probe, die ein repräsentatives Set von Antikörpern des Patienten enthält, der bevorzugt ein Säugetier, noch bevorzugter ein Mensch ist. Bevorzugt ist sie aus der Gruppe ausgewählt, die Serum, Plasma, Speichel und CSF umfasst.

Die erfindungsgemäße Lehre kann nicht nur unter Verwendung der Wildtypsequenzen der angegebenen Polypeptide oder der Referenzsequenzen, wie sie in Form von SEQ ID NOs, zusammen von nun an als "Volllängen-Polypeptid" bezeichnet, oder Datenbankencodes oder in sonstiger Weise, ggf. implizit, hier angegeben sind, ausgeführt werden, sondern auch unter Verwendung von Varianten dieser Sequenzen.

In einer besonders bevorzugten Ausführungsform bedeutet der Begriff "Variante", wie hierin verwendet, dabei wenigstens ein Fragment des Volllängen-Polypeptids, das am C- und/oder N-terminalen Ende gegenüber dem Volllängen-Polypeptid um eine oder mehr als eine Aminosäure verkürzt ist. Ein solche Fragment kann eine Länge von 5, 6, 7, 8, 10, 12, 15, 20, 25, 50, 75, 100, 150 oder, 200 aufeinanderfolgenden Aminosäuren aus der Sequenz des Volllängen-Polypeptides enthalten, bevorzugt 10 oder mehr.

In einer weiteren bevorzugten Ausführungsform umfasst der Begriff "Variante", wie hierin verwendet, nicht nur ein Fragment, sondern auch ein Polypeptid, das Aminosäuresequenzen mit in der Reihenfolge zunehmender Bevorzugung wenigstens 40, 50, 60, 70, 75, 80, 85, 90, 92, 94, 95, 96, 97, 98 oder 99 % Sequenzidentität zum Volllängen-Polypeptid oder zum Fragment davon aufweist, wobei keine für die biologische Aktivität, z.B. die Fähigkeit eines Antigens, an einen für das Volllängen-Polypeptid-Antigen spezifischen Antikörper zu binden, essentielle Aminosäure deletiert oder nicht-konservativ substituiert wird. Der Stand der Technik offenbart Verfahren zur Feststellung der Sequenzidentität von Aminosäuresequenzen, z.B. in Arthur Lesk (2008), Introduction to bioinformatics, Oxford University Press, 2008, dritte Ausgabe. In einer bevorzugten Ausführungsform wird die ClustalW-Software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) unter Verwendung der Standardeinstellungen dazu benutzt.

Zusätzlich kann ein erfindungsgemäß verwendetes Polypeptid oder eine Variante davon chemische Modifikationen aufweisen, z.B. Isotopenlabel, kovalente Modifikationen wie Glykosylierung, Phosphorylierung, Acetylierung, Decarboxylierung, Citrullinierung, Methylierung, Hydroxylierung usw. In einer besonders bevorzugten Ausführungsform ist ein zum Nachweis eines Autoantikörpers gegen die beta-Untereinheit oder sonst erfindungsgemäß verwendetes Polypeptid umfassend die beta-Untereinheit oder eine Variante davon glykosyliert. Die Glykosylierung wird bevorzugt durch Expression in einer zur Glykosylierung befähigten Zelle bewerkstelligt, bevorzugt eine Säugetier- oder Insektenzelle, bevorzugter eine Säugetierzelle.

Varianten können auch Fusionsproteine mit Aminosäuren oder anderen bekannten Polypeptiden oder Varianten davon sein, bevorzugt mit einem Tag zur Aufreinigung, noch bevorzugter aus der Gruppe umfassend His Tag, Thioredoxin, Maltose-bindendes Protein, Streptavidin, Glutathion-S-Transferase oder einer Variante davon, und aktive Teile oder Domänen sein oder aufweisen, bevorzugt mit einer Sequenzidentität von wenigstens 70, 75, 80, 85, 90, 92, 94, 95, 96, 97, 98 oder 99 % zum Volllängen-Polypeptid. In einer bevorzugten Ausführungsform wird unter dem Begriff "aktive Teile oder Domänen" ein Fragment des Volllängen-Polypeptids oder eine Variante verstanden, das bzw. die wenigstens einen Teil der biologischen Aktivität des Volllängen-Polypeptids behält.

Es ist essentiell, dass die Variante des Volllängen-Polypeptids biologische Aktivität aufweist. In einer bevorzugten Ausführungsform handelt es sich bei der biologischen Aktivität um die Fähigkeit eines Mittels, bevorzugt Polypeptides, einen Autoantikörper gegen die beta-Untereinheit einzufangen oder daran zu binden, bevorzugt spezifisch zu binden, wie er in Proben von Patienten vorkommt, die an AIG erkrankt sind. Als Referenz für Versuche kann der Autoantikörper aus solchen Proben gewonnen werden. Varianten eines sekundären Antikörpers, der zum Nachweis eines spezifisch eingefangenen Antikörpers dient, sind sämtliche Moleküle, die die gleiche Bindungsspezifität wie der sekundäre Antikörper aufweisen, bevorzugt die Fähigkeit, gegen alle Antikörper der menschlichen Klasse IgG zu binden. Sie umfassen beispielsweise Fragmente und Derivate des sekundären Antikörpers. Bei der Herstellung von Varianten oder der Einschätzung, ob diese die biologische Aktivität zeigen können, kann sich der Fachmann an den Arbeiten von da Silva *et al.* orientieren (da Silva, H. D., Gleeson, P. A., Toh, B. H., Van Driel, I. R. und Carbone, F. R. (1999) Identification of a gastritogenic epitope of the H/K ATPase beta-subunit, Immunology 1999, 96, 145-151). Sie identifizierten SEQ ID NO6 als Peptid, das das immunodominante Epitop enthält. Das bedeutet, dass die homologe Sequenz aus dem humanen Protein, dargestellt in SEQ ID NO5, das humane Epitop enthält. Durch Sequenzalignments mit weiteren Säugetier-Homologen, z.B. aus dem Schwein, Rind, aus Primaten, Ziege und weiteren kann der Fachman leicht reaktive Varianten vorhersagen. Durch Mutationen und weitere Trunkierungen kann der Fachmann weitere Varianten generieren. Mittels ELISA ausgeführt wie in den Beispielen kann er bestätigen, dass die biologische Aktivität erhalten bleibt.

Dabei bindet ein Polypeptid oder eine Variante davon nicht oder nur in geringem Maß gegen Antikörper gegen die alpha-Untereinheit, bevorzugt deren immunodominantes Epitop, wie es in SEQ ID NO8 wiedergegeben ist. Homologe Epitope aus weiteren homologen Säugetier-Enzymen kann der Fachmann aus Nishio et al. entnehmen und für das Design von Varianten berücksichtigen (Nishio, A., Hosono, M., Watanabe, Y., Sakai, M., Okuma, M und Masuda, T (1994) Gastroenterology 107, 1408-1414). Bevorzugt ist die Bindungskonstante von Autoantikörpern gegen die beta-Untereinheit, wie sie in einer Probe von einem Patienten vorkommen, wenigstens 10, bevorzugt, 100, bevorzugter 1.000, bevorzugter 10.000, bevorzugter 100.000, noch bevorzugter 1.000.000, noch bevorzugter 10.000.000 fach niedriger und die Stärke der Bindungsreaktion entsprechend höher als die Bindungskonstante von Autoantikörpern gegen die alpha-Untereinheit, wie sie in einer Probe von einem Patienten vorkommen. Dabei charakterisiert die Bindungskonstante die Stärke der Bindung des jeweiligen Autoantikörpers gegen die Variante, genauer einer Mischung der Autoantikörper, wie sie in einer Probe vorkommen. Die Bindungskonstante wird dabei bevorzugt mittels Biacore in PBS-Puffer bei 25 °C gemessen. Zum Beispiel kann die Bindungskonstante bei der Bindung von Autoantikörpern gegen die beta-Untereinheit aus einer Probe 10⁻¹⁰ M betragen, während die Bindungskonstante der identischen Autoantikörper gegen die alpha-Untereinheit aus der Probe 10⁻⁶ M beträgt. Die erste Bindungskonstante ist damit 10.000-fach niedriger als die zweite. Das bedeutet, dass Autoantikörper gegen die beta-Untereinheit deutlich stärker an das Polypeptid oder die Variante binden als Autoantikörper gegen die alpha-Untereinheit. Wird das Polypeptid oder die Variante erfindungsgemäß eingesetzt, so werden damit lediglich Autoantikörper gegen die beta-Untereinheit gebunden und nachgewiesen, nicht aber Autoantikörper gegen die alpha-Untereinheit.

In einer bevorzugten Ausführungsform wird unter dem Begriff "Autoantikörper", wie hierin verwendet, ein Antikörper verstanden, der spezifisch an eine Struktur, bevorzugt ein Autoantigen, aus dem Organismus bindet, welcher ihn herstellt. Ein solcher Autoantikörper weist eine konstante Region auf, wie sie andere Antikörper der gleichen Klasse aus dem gleichen Organismus aufweisen. Besonders bevorzugt handelt es sich um einen Säugetier-, noch bevorzugter um einen menschlichen Autoantikörper, noch bevorzugter um einen menschlichen Autoantikörper der Klasse IgG. Die variable Domäne ist in der Lage gegen das Autoantigen spezifisch zu binden. Bevorzugt ist das Autoantigen ein Epitop abgeleitet von oder enthalten in der beta-Untereinheit, bevorzugt SEQ ID NO5 oder einer Variante davon.

In einer bevorzugten Ausführungsform ist das Mittel zum Einfangen ein isoliertes und/oder aufgereinigtes Polypeptid, das besonders bevorzugt rekombinant ist. Unter einem "isolierten" Polypeptid kann dabei verstanden werden, dass das Polypeptid aus seiner natürlichen Umgebung bzw. der Umgebung, in der es exprimiert wurde, entfernt wurde. Unter einem "aufgereinigten" Polypeptid wird in einer bevorzugten Ausführungsform ein Polypeptid verstanden, das unter Verwendung von chromatographischen Verfahren gegenüber der Konzentration und/oder Reinheit in seiner natürlichen Umgebung angereichert wurde, die bevorzugt aus der Gruppe ausgewählt sind, die Affinitätschromatographie, Gelfiltrationschromatographie und lonenaustausch-chromatographie umfasst. In einer besonders bevorzugten Ausführungsform ist ein Polypeptid aufgereinigt, wenn es eine Reinheit von wenigstens 40, 50, 60, 70, 80, 90, 95, 98, 99 oder 99,5% hat, was visuell oder, bevorzugt, durch Intensitätsmessung von Banden auf einem SDS-PAGE-Gel nach Coomassie-Färbung beurteilt werden kann.

Ein Polypeptid kann in Form eines Gewebes oder einer Zelle bereitgestellt werden, bevorzugt ein rekombinantes Gewebe oder eine rekombinante Zelle. Die Zelle ist bevorzugt eine eukaryontische Zelle, bevorzugter eine Insekten-, Pflanzen- oder Säugetierzelle, noch bevorzugter eine Säugetierzelle wie eine menschliche Zelle, am bevorzugtesten eine HEK293-Zelle.

Ein Polypeptid als Mittel kann in gefalteter Form oder in ungefalteter Form bereitgestellt werden und ist bevorzugt gefaltet. Bevorzugt wird die Faltung mittels CD-Spektroskopie in einem Puffer gemessen, der die Messung gestattet und in dem das Protein seine dreidimensionale Faltung annehmen kann (siehe beispielsweise Banaszak L. J. (2008), Foundations of Structural Biology, Academics Press, or Teng Q. (2013), Structural Biology: Practical Applications, Springer), z.B. in 10 mM Natriumphosphat bei pH 7. Bevorzugt nimmt das Protein eine Faltung an, die von einem nachzuweisenden Antikörper erkannt wird.

In einer bevorzugten Ausführungsform wird ein Antikörper nach dem Einfangen durch eine Methode aus der Gruppe nachgewiesen, die die Messung von Immundiffusion, Immunoelektrophorese, Lichtstreuung, Agglutination, Radioaktivität, enzymatische Aktivität, (Elektro)-Chemolumineszenz und Immunfluoreszenz umfasst. Im Falle einer Detektion durch Radioaktivität, enzymatische Aktivität, (Elektro-)Chemolumineszenz und Immunfluoreszenz kann eine detektierbare Markierung eingesetzt werden. Sie ist bevorzugt an einem sekundären Antikörper angebracht, der an den nachzuweisenden Antikörper bindet. Nachweismethoden sind beispielsweise in Zane, H. D. (2001), Immunology - Theoretical & Practical Concepts in Laboratory Medicine, W. B. Saunders Company, besonders in Kapitel 14 beschrieben.

Erfindungsgemäß wird ein Kit bereitgestellt, der den erfindungsgemäßen Träger umfasst, wobei der Kit ein oder mehr als ein Reagenz, bevorzugt alle Reagenzien, aus der Gruppe enthält, die eine Waschlösung, wenigstens eine Kalibratorlösung, einen Antikörper gegen die beta-Untereinheit der gastritischen H⁺/K⁺-ATPase, und ein Mittel zum Detektieren des Autoantikörpers, bevorzugt einen sekundären Antikörper, noch bevorzugter einen sekundären Antikörper gegen IgG-Antikörper oder die beta-Untereinheit der gastritischen H⁺/K⁺-ATPase oder eine Variante davon, bevorzugt mit nachweisbarer Markierung, einen Verdünnungspuffer zum Verdünnen der Probe, eine Positivkontrolle, eine Chemilumineszenz-Triggerlösung und ein chemilumineszenzfähiges Substrat umfasst. Statt der Chemilumineszenz-Triggerlösung und dem chemilumineszenzfähigen Substrat kann auch eine Chromogenlösung für einen ELISA enthalten sein.

Der Autoantikörper-Nachweis kann quantitativ oder semiquantitativ sein, bevorzugt semiquantitativ. In einer bevorzugten Ausführungsform versteht man, dass "semiquantitativ" bedeutet, dass bestimmt wird, ob das Messergebnis über einem bestimmten Schwellenwert liegt, z.B. 5,6,7,8,9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39 oder 40 RU/ml. Der Wert in RU/ml wird dabei bevorzugt mittels ELISA (EA 1361-9601 G) der EUROIMMUN Medizinische Labordiagnostika AG gemessen.

Unter einer Kalibratorlösung, wie sie beim erfindungsgemäßen Verfahren eingesetzt wird, wird eine Lösung mit einer definierten Menge eines Antikörpers verstanden, der gegen das gleiche Antigen bindet wie der nachzuweisende Autoantikörper und bevorzugt zur gleichen Ig-Klasse gehört wie der nachzuweisende Antikörper, wobei das Antigen bevorzugt die beta-Untereinheit ist. Der Kalibrator kann eine in absoluter oder relativem Maßstab bekannte Menge des Antikörpers enthalten. Z.B. kann ein Kalibrator zehn Mal so viel Antikörper enthalten wie ein anderer. Bei dem in definierter Menge vorhandenen Antikörper kann es sich um einen Autoantikörper aus einer Probe handeln oder um einen rekombinanten, monoklonalen Antikörper. Bevorzugt werden mehr als zwei, bevorzugt 3, 4, 5 oder 6 Kalibratorlösungen mit jeweils unterschiedlichen Konzentrationen des Antikörpers in das Kit gegeben oder erfindungsgemäß verwendet, insbesondere parallel zu einer Probe untersucht, so dass eine Kalibrationskurve zur semi-quantitativen oder quantitativen Bestimmung der Autoantikörpers gegen die beta-Untereinheit aufgezeichnet werden kann. In einer bevorzugten Ausführungsform umfasst der Kalibrator einen Autoantikörper oder Antikörper gegen die beta-Untereinheit der gastritischen H⁺/K⁺-ATPase, bevorzugt hingegen weniger oder nicht in detektierbaren Mengen oder keinen Autoantikörper oder Antikörper gegen die alpha-Untereinheit.

Erfindungsgemäß wird ein monoklonaler Antikörper oder isolierter Autoantikörper, der spezifisch gegen die beta-Untereinheit der gastritischen H⁺/K⁺-ATPase bindet, besonders bevorzugt in rekombinanter, isolierter und/oder aufgereinigter Form verwendet, am bevorzugtesten gegen SEQ ID NO5. Ein solcher Antikörper kann als Positivkontrolle für erfindungsgemäße Verfahren dienen sowie als Reagenz für die Immobilisierung der beta-Untereinheit oder als Reagenz für kompetitive Testformate, für die er optional eine nachweisbare Markierung aufweisen kann.

In einer bevorzugten Ausführungsform wird der eingefangene Autoantikörper mittels Chemilumineszenz nachgewiesen. Zu diesem Zweck kann der Autoantikörper mit einem chemilumineszenzfähigen Liganden kontaktiert werden, der an den Autoantikörper bindet. Ein chemilumineszenzfähiger Ligand kann ein sekundärer Antikörper sein, der an die konstante Domäne des Autoantikörpers bindet, oder die beta-Untereinheit der gastritischen H⁺/K⁺-ATPase oder eine Variante davon, die an die variable Domäne bindet. Der chemilumineszenzfähige Ligand kann ein chemilumineszenzfähiges Enzym sein, beispielsweise Luciferase, Peroxidase, alkalische Phosphatase und β-Galaktosidase, welches ein chemilumineszenzfähiges Substrat umsetzen kann ohne dabei verbraucht zu werden (Kricka, L. J. (2003).Clinical applications of chemiluminescence. Analytica chimica acta, 500(1): 279-286). Der chemilumineszenzfähige Ligand kann aber auch eine chemilumineszenzfähige organische Verbindung ohne enzymatische Aktivität aufweisen, die in Gegenwart einer Chemilumineszenz-Triggerlösung ein Chemilumineszenz-Signal aussendet, beispielsweise einen Acridinium-Ester (Weeks, I., Beheshti, I., McCapra, F., Campbell, A. K., Woodhead, J. S., Acridinium esters as high specific activity labels in immunoassay. Clin Chem 29: 1474-1479 (1983)) oder Luminol oder ein chemilumineszenzfähiges Derivat davon. Diese Verbindung wird bei der Reaktion verbraucht.

Im Falle eines Acridiniumesters wird als Triggerlösung eine Mischung aus Hydrogenperoxid und Hydroxid, z.B. in Form von Natriumhydroxid eingesetzt. Die Acridiniumester-Verbindung wird dabei unter alkalischen Bedingungen oxidiert, was Licht-emittierendes Acridon erzeugt. Das Signal wird in Form eines Lichtblitzes (Flash-Lumineszenz), welcher typischerweise 1-5 sec andauert, freigesetzt. Die kurze Dauer der Emission erfordert die Initiierung und Messung der Reaktion direkt am Detektor.

Bevorzugt wird die Chemilumineszenz als Nachweismethode mit einem Träger eingesetzt, bei dem es sich um einen Bead handelt.

In einer weiteren bevorzugten Ausführungsform wird der eingefangene Autoantikörper mittels ELISA nachgewiesen. Zu diesem Zweck wird das Mittel zum spezifischen Einfangen in wenigstens einem, bevorzugt mehreren, bevorzugt wenigstens 2, 4, 6, 8, 10, 12, 16, 20 oder 24 Wells einer Mikrotiterplatte für ELISA immobilisiert. In wenigstens ein Well wird eine zu analysierende Probe gegeben, in andere Wells werden 2, 3, 4, 5 oder mehr Kalibratorlösungen gegeben. Der eingefangene Antikörper wird dann bevorzugt über einen sekundären Antikörper mit einer enzymatisch aktiven Markierung nachgewiesen.

Erfindungsgemäß wird ein Mittel zum spezifischen Einfangen eines Autoantikörpers gegen die beta-Untereinheit der gastritischen H⁺/K⁺-ATPase zum Kalibrieren oder zur Kontrolle der Qualität eines Medizinproduktes verwendet. In einer bevorzugten Ausführungsform wird unter "Kalibrieren" verstanden, dass unter Verwendung von Kalibrationslösungen Messwerte erhalten werden, die bestätigen, dass das Gerät in einem erforderlichen Konzentrationsbereich zuverlässige Messwerte liefert oder eine zuverlässige Abgrenzung zwischen der Anwesenheit oder Abwesenheit des Autoantikörpers in einer Probe gestattet. In einer weiteren bevorzugten Ausführungsform kann "Kontrolle" bedeuten, dass verifiziert wird, dass das Gerät in dem Sinne zuverlässig arbeitet, dass eine Probe enthaltend den Autoantikörper tatsächlich als positiv gemessen wird und eine Probe ohne den Autoantikörper tatsächlich als negativ gemessen wird. In einer weiteren Ausführungsform bedeutet "Kontrolle", dass überprüft wird, ob eine Charge des Mittels oder ein Gerät unter gegebenen Bedingungen die Fähigkeit, bevorzugt noch genügend Kapazität besitzt, um den Autoantikörper zu binden. Bei dem Medizinprodukt kann es sich um ein diagnostisches Produkt wie den erfindungsgemäßen Träger handeln. Alternativ kann es sich um eine therapeutische Vorrichtung handeln, beispielsweise für Apherese, die ebenfalls ein Mittel zum spezifischen Einfangen oder Nachweisen des Autoantikörpers umfasst.

In einer bevorzugten Ausführungsform stellt die Erfindung eine Verwendung oder ein Reagenz oder Mittel oder Polypeptid zum Nachweisen eines Autoantikörpers gegen die beta-Untereinheit dar, oder eine Verwendung einer Nukleinsäure kodiferend die beta-Untereinheit oder eine Variante davon oder eine Nukleinsäure, die unter stringenten Bedinungen an diese Nukleinsäure hybridisiert oder einen Vektor oder eine Zelle umfassend die Nukleinsäure oder den Vektor zur Herstellung eines Kits zur Diagnose einer Krankheit. In einer bevorzugten Ausführungsform wird eine Zelle umfassend den Vektor, und bevorzugt nicht umfassend einen Vektor kodierend für die alpha-Untereinheit oder ein Epitop oder eine Variante davon, bereitgestellt und unter Bedingungen kultiviert, die das Exprimieren der Nukleinsäure gestatten, gefolgt vom Isolieren und/oder Aufreinigen des Expressionsproduktes, gefolgt vom Verwenden des Expressionsproduktes als Mittel zum spezifischen Einfangen eines Antikörpers in einem Verfahren oder Produkt gemäß der vorliegenden Erfindung.

In einer bevorzugten Ausführungsform stellt die vorliegende Erfindung einen Apparat zum Analysieren einer Probe von einem Patienten zum Nachweisen von einem Autoantikörper gegen die beta-Untereinheit bereit, wobei der Nachweis eine erhöhte Warhscheinlichkeit einer Krankheit, bevorzugt AIG und/oder PAG, anzeigt, wobei der Apparat umfasst:
a. einen Träger, der ein Mittel zum spezifischen Einfangen des Autoantikörpers umfasst, wenn eine Probe mit dem Träger kontaktiert wird,
b. ein nachweisbares Mittel, das in der Lage ist, an den Antikörper zu binden, wenn dieser gemäß a. beim Kontaktieren von Träger und Probe eingefangen wurde, wobei das Mittel insbesondere ein markierter sekundärer Antikörper ist, der an den eingefangenen Antikörper bindet,
c. eine Detektierungvorrichtung zum Nachweisen der Anwesenheit des nachweisbaren Mittels und zum Umwandeln des dabei erhaltenen Ergebnisses in ein Signal, bevorzugt ein elektrisches Signal, und
d. optional ein Mittel zum Empfangen des Signals aus der Detektierungsvorrichtung und zum Bestimmen, ob die Höhe des Signals eine erhöhte Wahrscheinlichkeit der Krankheit anzeigt, genauer durch Vergleichen der Höhe des Signals mit der Höhe eines Hintergrund- oder Schwellensignals oder einem eingegebenen Signalwert, das mit Proben von gesunden Personen generiert wurde.

In der vorliegenden Patentanmeldung sind neue Polypeptide und/oder Nukleinsäuren aufgeführt. Sie haben die folgenden Sequenzen: 
SEQ ID NO1: spGh*-H6-ATP4B(ec)
MKHLWFFLLLVAAPRWVLSGPMHHHHHHMSQTVDPYTPDYQDQLRSPGVTLRPDVYGEK
GLEIVYNVSDNRTWADLTQTLHAFLAGYSPAAQEDSINCTSEQYFFQESFRAPNHTKFSCKF
TADMLQNCSGLADPNFGFEEGKPCFIIKMNRIVKFLPSNGSAPRVDCAFLDQPRELGQPLQ
VKYYPPNGTFSLHYFPYYGKKAQPHYSNPLVAAKLLNIPRNAEVAIVCKVMAEHVTFNNPHD
PYEGKVEFKLKIEK
 
SEQ ID NO2: H6-ATP4B(ec); tatsächlich in der Zelle vorhandene und für die Beispiele
aufgereinigte Sequenz, d.h. extrazelluläre Domäne, mit N-terminalem His-Tag
GPMHHHHHHMSQTVDPYTPDYQDQLRSPGVTLRPDVYGEKGLEIVYNVSDNRTWADLTQ
TLHAFLAGYSPAAQEDSINCTSEQYFFQESFRAPNHTKFSCKFTADMLQNCSGLADPNFGF
EEGKPCFIIKMNRIVKFLPSNGSAPRVDCAFLDQPRELGQPLQVKYYPPNGTFSLHYFPYYG
KKAQPHYSNPLVAAKLLNIPRNAEVAIVCKVMAEHVTFNNPHDPYEGKVEFKLKIEK
 
SEQ ID NO3: Extrazelluläre Domäne der ATP4B-Untereinheit
QTVDPYTPDYQDQLRSPGVTLRPDVYGEKGLEIVYNVSDNRTWADLTQTLHAFLAGYSPAA
QEDSINCTSEQYFFQESFRAPNHTKFSCKFTADMLQNCSGLADPNFGFEEGKPCFIIKMNRI
VKFLPSNGSAPRVDCAFLDQPRELGQPLQVKYYPPNGTFSLHYFPYYGKKAQPHYSNPLVA
AKLLNIPRNAEVAIVCKVMAEHVTFNNPHDPYEGKVEFKLKIEK
**SEQ ID NO4: Transmembrandomäne der ATP4B-Untereinheit**
   SLYYVAFYVVMTGLFALCLYVLM
**SEQ ID NO5: Immunodominantes Epitop der humanen ATP4B-Untereinheit homolog**
   **zu dem aus da Silva *et al.***
   LLNIPRNAEVAIVCKVMAEHVTFNN
**SEQ ID NO6: Immunodominantes Epitop der murinen ATP4B-Untereinheit gemäß da Silva *et al.***
   LLNVPKNMQVSIVCKILADHVTFNN 
SEQ ID NO7: in P51164 dargestellte Aminosäuresequenz der ATP4B-Untereinheit
MAALQEKKTCGQRMEEFQRYCWNPDTGQMLGRTLSRWVWISLYYVAFYVVMTGLFALCL
YVLMQTVDPYTPDYQDQLRSPGVTLRPDVYGEKGLEIVYNVSDNRTWADLTQTLHAFLAGY
SPAAQEDSINCTSEQYFFQESFRAPNHTKFSCKFTADMLQNCSGLADPNFGFEEGKPCFIIK
MNRIVKFLPSNGSAPRVDCAFLDQPRELGQPLQVKYYPPNGTFSLHYFPYYGKKAQPHYSN
PLVAAKLLNIPRNAEVAIVCKVMAEHVTFNNPHDPYEGKVEFKLKIEK
**SEQ ID NO8: Immunodominantes Epitop der murinen ATP4A-Untereinheit gemäß Nishio et al.**
   PLLCVGLRAQWEDHHLQDL 
SEQ ID NO9: alpha-Untereinheit der gastritischen H+/K+-ATPase, wie in UniProtKB-
Zugriffsnummer P20648 dargestellt
MGKAENYELYSVELGPGPGGDMAAKMSKKKKAGGGGGKRKEKLENMKKEMEINDHQLSV
AELEQKYQTSATKGLSASLAAELLLRDGPNALRPPRGTPEYVKFARQLAGGLQCLMWVAAA
ICLIAFAIQASEGDLTTDDNLYLAIALIAVVVVTGCFGYYQEFKSTNIIASFKNLVPQQATVIRDG
DKFQINADQLVVGDLVEMKGGDRVPADIRILAAQGCKVDNSSLTGESEPQTRSPECTHESP
LETRNIAFFSTMCLEGTVQGLVVNTGDRTIIGRIASLASGVENEKTPIAIEIEHFVDIIAGLAILFG
 
ATFFIVAMCIGYTFLRAMVFFMAIVVAYVPEGLLATVTVCLSLTAKRLASKNCVVKNLEAVETL
GSTSVICSDKTGTLTQNRMTVSHLWFDNHIHTADTTEDQSGQTFDQSSETWRALCRVLTLC
NRAAFKSGQDAVPVPKRIVIGDASETALLKFSELTLGNAMGYRDRFPKVCEIPFNSTNKFQL
SIHTLEDPRDPRHLLVMKGAPERVLERCSSILIKGQELPLDEQWREAFQTAYLSLGGLGERV
LGFCQLYLNEKDYPPGYAFDVEAMNFPSSGLCFAGLVSMIDPPRATVPDAVLKCRTAGIRVI
MVTGDHPITAKAIAASVGIISEGSETVEDIAARLRVPVDQVNRKDARACVINGMQLKDMDPSE
LVEALRTHPEMVFARTSPQQKLVIVESCQRLGAIVAVTGDGVNDSPALKKADIGVAMGIAGS
DAAKNAADMILLDDNFASIVTGVEQGRLIFDNLKKSIAYTLTKNIPELTPYLIYITVSVPLPLGCI
TILFIELCTDIFPSVSLAYEKAESDIMHLRPRNPKRDRLVNEPLAAYSYFQIGAIQSFAGFTDYF
TAMAQEGWFPLLCVGLRAQWEDHHLQDLQDSYGQEWTFGQRLYQQYTCYTVFFISIEVCQ
IADVLIRKTRRLSAFQQGFFRNKILVIAIVFQVCIGCFLCYCPGMPNIFNFMPIRFQWWLVPLP
YGILIFVYDEIRKLGVRCCPGSWWDQELYY
 
SEQ ID NO10: Intrinsic factor, wie in NCBI-Genbankeintrag NP_005133.2 dargestellt
MAWFALYLLSLLWATAGTSTQTQSSCSVPSAQEPLVNGIQVLMENSVTSSAYPNPSILIAMN
LAGAYNLKAQKLLTYQLMSSDNNDLTIGQLGLTIMALTSSCRDPGDKVSILQRQMENWAPSS
PNAEASAFYGPSLAILALCQKNSEATLPIAVRFAKTLLANSSPFNVDTGAMATLALTCMYNKI
PVGSEEGYRSLFGQVLKDIVEKISMKIKDNGIIGDIYSTGLAMQALSVTPEPSKKEWNCKKTT
DMILNEIKQGKFHNPMSAIQILPSLKGKTYLDVPQVTCSPDHEVQPTLPSNPGPGPTSASNIT
VIYTINNQLRGVELLFNETINVSVKSGSVLLVVLEEAQRKNPMFKFETTMTSWGLVVSSINNIA
ENVNHKTYWQFLSGVTPLNEGVADYIPFNHEHITANFTQY
**SEQ ID** N011: **cDNA kodierend für die ATP4A-Untereinheit ligiert in den Vector pTriEx**
   [Nur im Sequenzprotokoll enthalten]
**SEQ ID NO12: cDNA kodierend für die ATP4B-Untereinheit ligiert in den Vector pTriEx**
   [Nur im Sequenzprotokoll enthalten]

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

**Fig. 1** zeigt ein Coomassie-gefärbtes Polyacrylamidgel, auf dem rechts neben dem Marker 1 µg der gereinigten extrazellulären Domäne der beta-Untereinheit der gastritischen H⁺/K⁺-ATPase mit N-terminalem His-Tag DTT reduziert und rechts daneben nicht reduziert aufgetrennt wurde.

**Fig. 2** zeigt die Probenverteilung zu den in Beispiel 3 (Sensitivität) und Tabelle 4 gezeigten Daten. Die Y-Achse zeigt das Signal in RLU, jede Zahl der X-Achse steht für einen Patienten, der unter AIG leidet (1-29) bzw. für einen gesunden Blutspender (ab 30).

### Beispiele:

Es wurden zur Untersuchung der Sensitivität Seren von Patienten eingesetzt, bei denen die Diagnose AIG gestellt wurde. Auf der anderen Seite wurden zur Untersuchung der Spezifität Seren von klinisch unauffälligen Blutspendern eingesetzt, bei denen davon auszugehen ist, dass sie nicht unter AIG leiden.

### Beispiel 1: Bestimmung der Sensitivität eines Nachweises mittels der beta-Untereinheit der gastritischen H⁺/K⁺-ATPase als Antigen unter Verwendung von Immunfluoreszenz und ELISA

Die humane mit Genbank-Zugriffsnummer BC167780 beschriebene cDNA, kodierend für die ATP4A-Untereinheit, wurde in den Vector pTriEx ligiert (ergab SEQ ID NO11). Die humane mit Genbank-Zugriffsnummer BC029059 beschriebene cDNA, kodierend für die ATP4B-Untereinheit, wurde ebenfalls in den Vector pTriEx ligiert (ergab SEQ ID NO12).

### Herstellung von Substraten für die indirekte Immunfluoreszenz

Auf Glasträgern in D-MEM mit einem Zusatz von 10% (v/v) fötalem Kälberserum kultivierte HEK293-Zellen wurden mittels 25 kDa linearem Polyethylenimin mit der für ATP4A bzw. ATP4B kodierenden Plasmid-DNA einzeln sowie in Kombination und zusätzlich zur Herstellung einer Negativkontrolle mit dem pTriEx-Leervector transfiziert und nach 48 Stunden fixiert. Zur Fixation wurden die Glasträger mit den darauf haftenden Zellen zunächst mit PBS gewaschen und anschließend alternativ für 5 Minuten mit 1,8% Formalinlösung in PBS bzw. für 10 Minuten in Aceton bei Raumtemperatur fixiert. Abschließend erfolgte ein Wasch- und Blockierungsschritt in PBS mit 1% (w/v) BSA Zusatz (PBS (1% w/v BSA)) für 5 Minuten bei Raumtemperatur.

Alle Inkubationsschritte wurden bei Raumtemperatur durchgeführt.

### Immunfluoreszenz-Analyse

Die zu analysierenden Serum- bzw. Plasmaproben wurden in einer 1:10-Verdünnung in PBS (1% w/v BSA) für 30 Minuten auf dem Substrat inkubiert. Anschließend wurden die Substrate in PBS (0,2% v/v Tween 20) für 5 Minuten gewaschen. Gebundenes humanes Immunglobulin der Klasse IgG wurde mit Fluoreszein-markiertem anti-human-IgG in einem 30 minütigen Inkubationsschritt detektiert. Überschüssiges Konjugat wurde vor der Auswertung am Immunfluoreszenzmikroskop für 5 Minuten mit PBS (0,2% v/v Tween 20) entfernt.

Die Auswertung der Immunfluoreszenz erfolgte semiquantitativ durch Angabe der Intensität des spezifischen Fluoreszenzsignals in Intensitäten von 0 (keine Fluoreszenz), Spur (grenzwertiges Signal), 1 (schwach positiv), 2, 3 und 4 (stark positiv).

### Aufreinigung der extrazellulären Domäne von ATP4B (SEQ ID NO2) exprimiert mit Vektor pTriEx-1 in HEK293-Zellen:

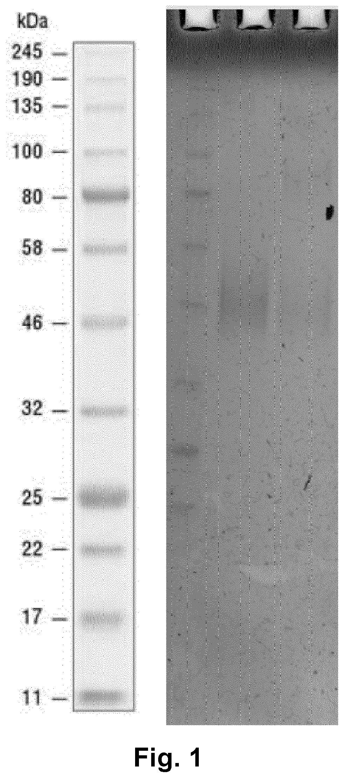

In D-MEM mit einem Zusatz von 10% (v/v) fötalem Kälberserum kultivierte HEK293-Zellen wurden mittels 25 kDa linearem Polyethylenimin mit der für spGh*-H6-ATP4B(ec)-kodierenden Plasmid-DNA transfiziert. Am Tag 5 nach Transfektion wurde der Zellkulturüberstand abgenommen, durch Zentrifugation geklärt und zur Reinigung des rekombinanten Proteins durch immobilisierte Metallchelat-Affinitätschromatografie (IMAC) unter Verwendung von Standardprotokollen eingesetzt. Das gereinigte, mit Ponceau S gefärbte Protein ist in **Fig. 1** gezeigt.

### Analyse mittels ELISA:

Die Versuche wurden durchgeführt wie beschrieben in Dähnrich et al. (2013) Development of a standardized ELISA for the determination of autoantibodies against human M-type phospholipase A2 receptor in primary membranous nephropathy, Clinica Chimica Acta 421, 213-218, abgesehen davon, dass als Antigen die extrazelluläre Domäne von ATP4B (SEQ ID NO2) eingesetzt wurde.

Kurz gesagt wurden Mikrotiterplatten (Nunc, Deutschland) mit 0,7 µg/ml Antigen in PBS über Nacht bei 4 °C beschichtet und drei Mal mit Waschpuffer (0,05% (w/v) Tween-20 in PBS) gewaschen. Unspezifische Bindestellen wurden durch einstündige Inkubation mit 0,1% Casein (w/v) in PBS blockiert.

Proben von menschlichem Serum wurden 1:100 in Probenpuffer (0,05% (w/v) Tween-20, 1% (w/v) Casein in PBS) verdünnt und 30 Minuten lang inkubiert, gefolgt von drei Waschschritten mit Waschpuffer. Neben zwei positiven Proben von klinisch charakterisierten Patienten wurden sieben weitere Proben von Patienten untersucht.

Gebundene Antikörper wurden durch eine halbstündige Inkubation mit anti-humanem-IgG HRP-Konjugat (EUROIMMUN, Deutschland) nachgewiesen, das 1:1000 in Probenpuffer verdünnt war. Anschließend wurde wie oben geschrieben gewaschen, gefolgt von Zugabe von 3,3',5,5'-Tetramethylbenzidin- (EUROIMMUN Medizinische Labordiagnostika AG) und fünfzehnminütiger Inkubation. Alle Inkubationsschritte wurden bei Raumtemperatur durchgeführt.

Die optische Dichte (OD) wurde bei 450 nm unter Verwendung eines automatischen Spektrophotometers (Spectra Mini, Tecan, Crailsheim, Deutschland) gemessen. Der Cut-off wurde durch ROC-Kurvenanalyse (maximale Summe der Sensitivität plus Spezifität) ermittelt.

Zum Vergleich wurde der Test auch unter Verwendung des herkömmlichen Anti-PCA ELISA (IgG) durchgeführt (EUROIMMUN Medizinische Labordiagnostika AG, Bestellnummer EA 1361 G).

### Ergebnisse:

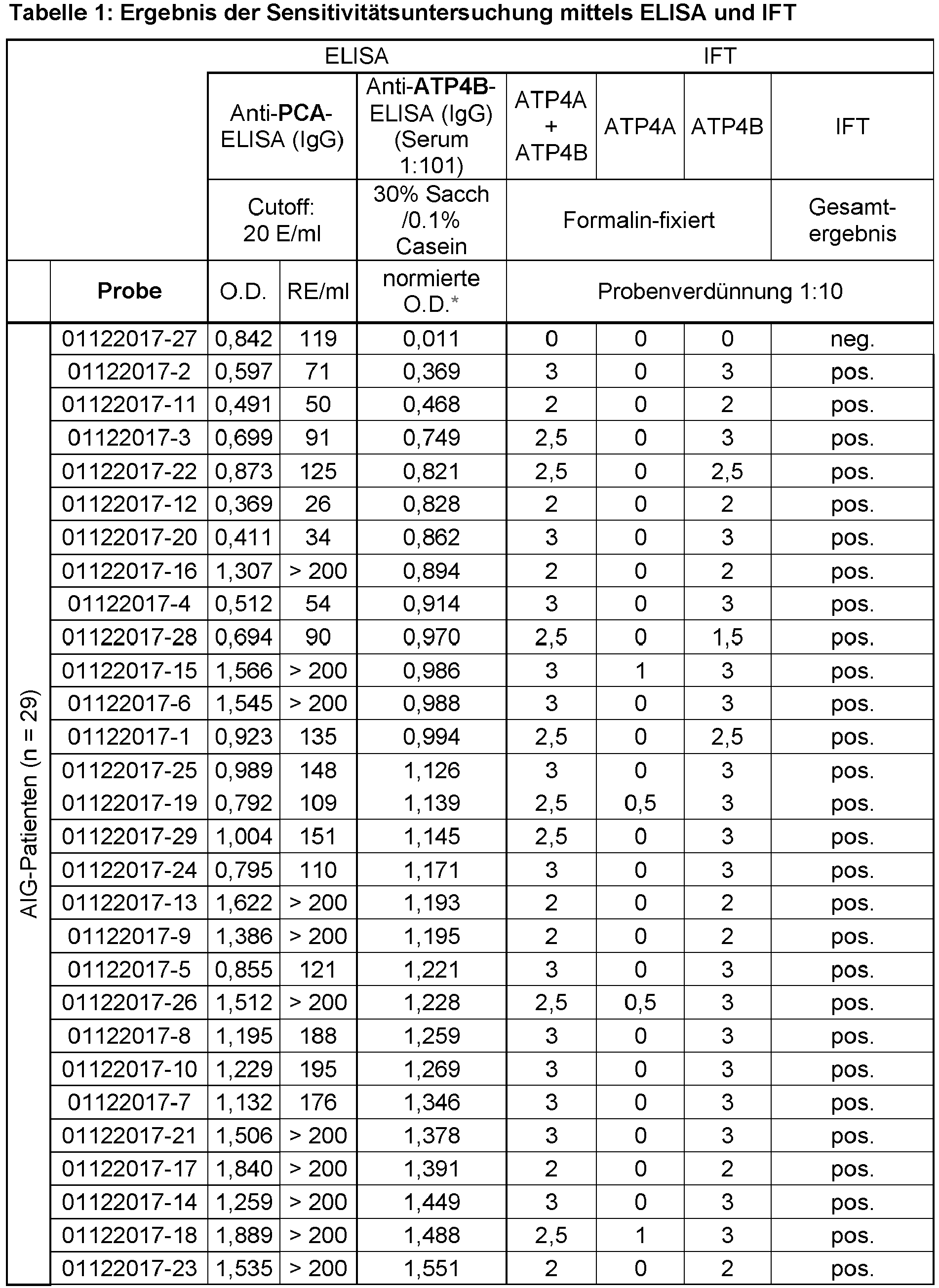

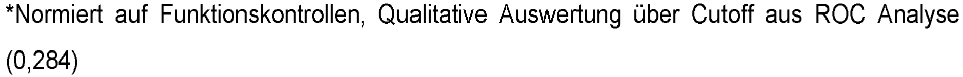

### Beispiel 2: Bestimmung der Spezifität eines Nachweises mittels der beta-Untereinheit der gastritischen H⁺/K⁺-ATPase als Antigen unter Verwendung von ELISA im Vergleich zum konventionellen Test (Stand der Technik):

Die Versuche wurden durchgeführt wie in Beispiel 1 beschrieben, abgesehen davon, dass Seren von klinisch unauffälligen Blutspendern eingesetzt wurden.

### Ergebnis:

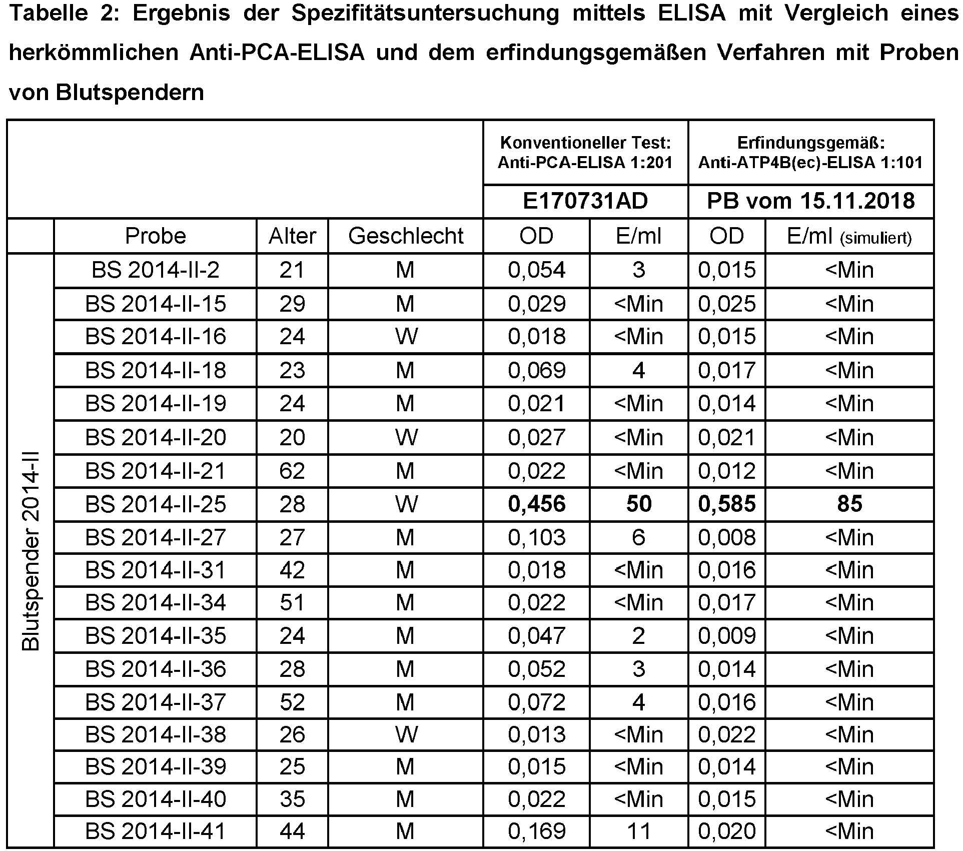

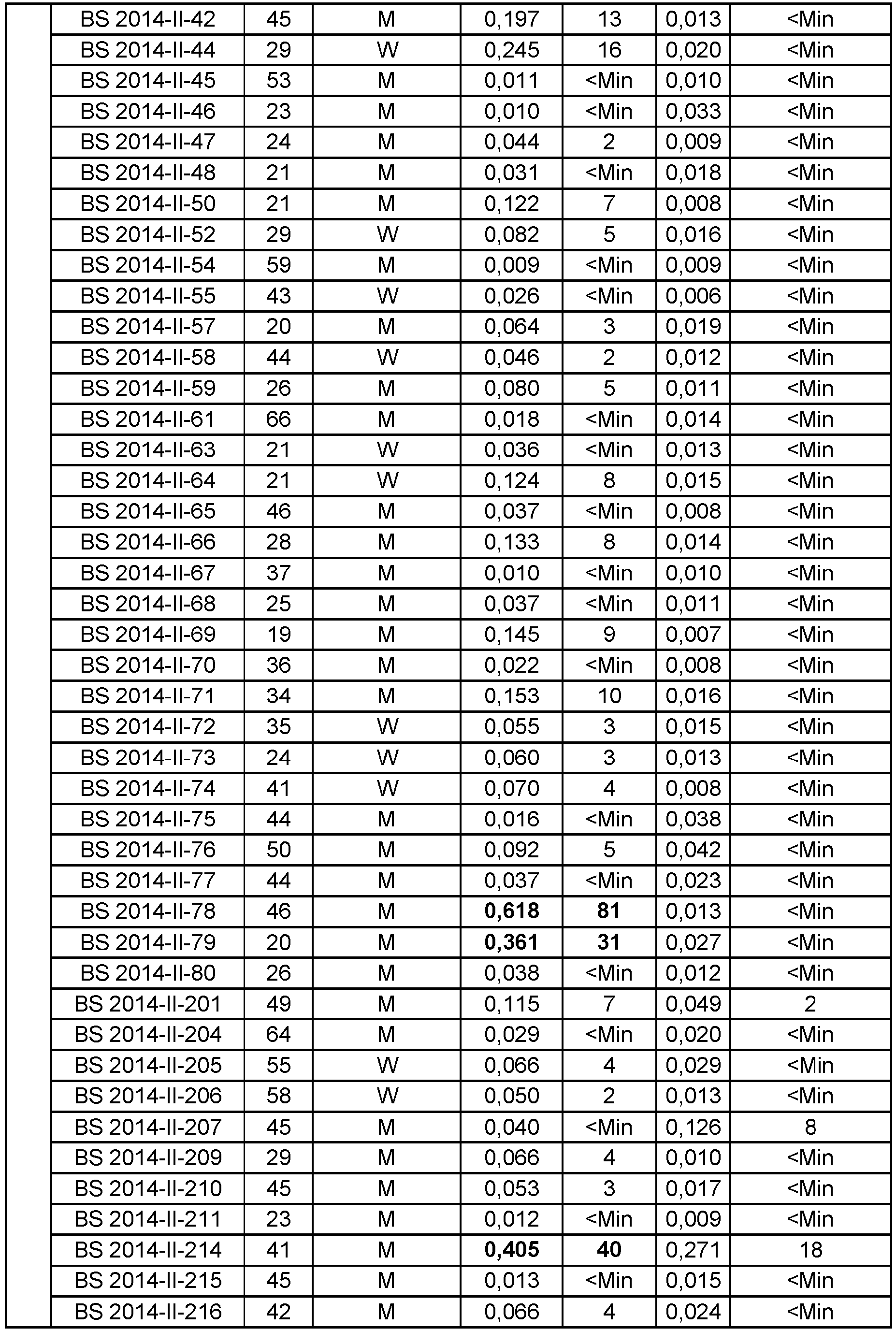

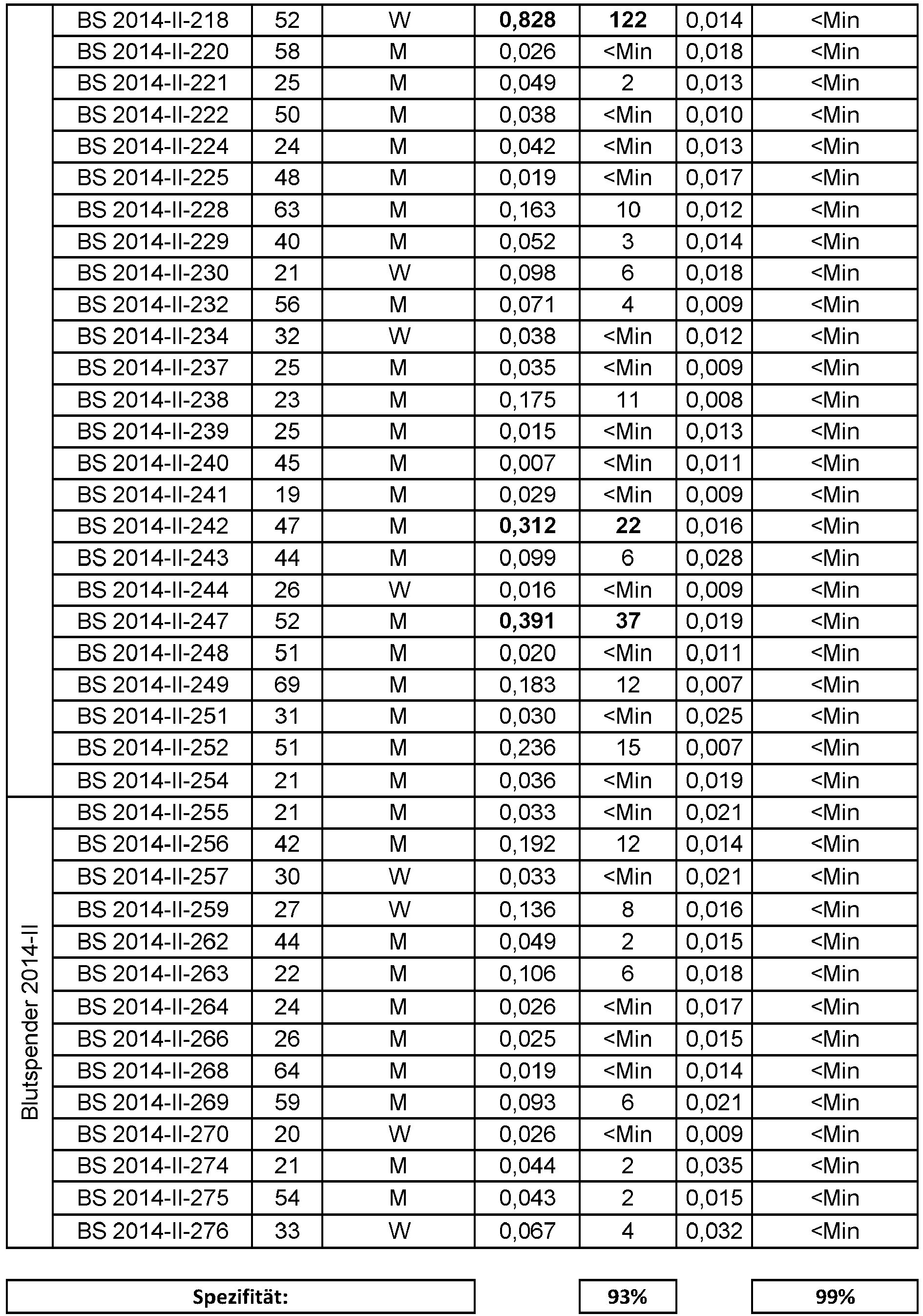

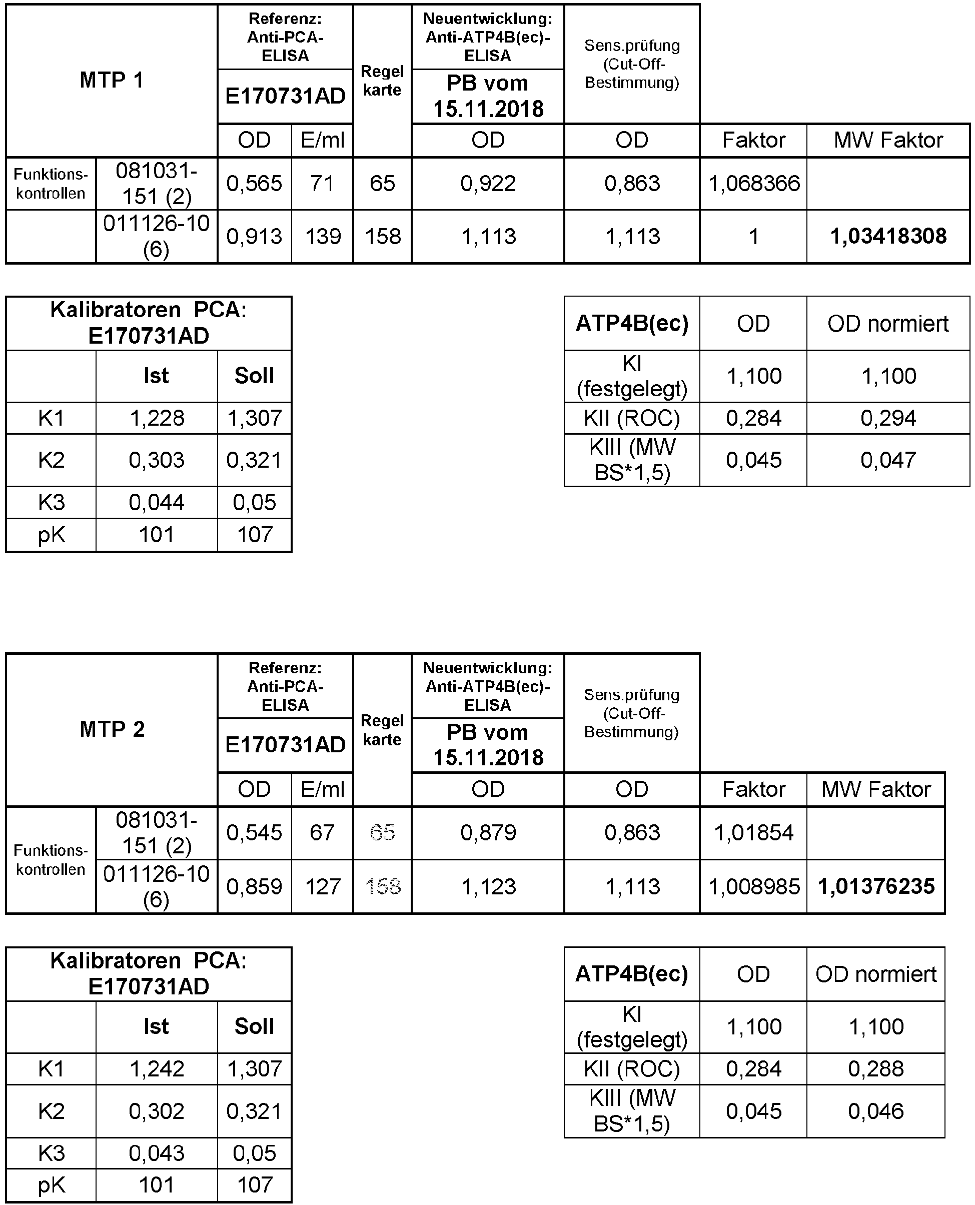

### Schlussfolgerung:

Die Ergebnisse zeigen, dass mit Ausnahme der Probe des erstgenannten Patienten auch dann der Autoantikörper gegen die gastritischen H⁺/K⁺-ATPase in den Proben der unter AIG leidenden Patienten nachgewiesen werden kann, wenn als Antigen lediglich die extrazelluläre Domäne der beta-Untereinheit der gastritischen H⁺/K⁺-ATPase verwendet wird.

Gegenüber dem konventionellen ELISA ergibt sich also praktisch kein Sensitivitätsverlust.

Unter Verwendung der alpha-Untereinheit der gastritischen H⁺/K⁺-ATPase erscheinen hingegen praktisch alle Proben als falsch negativ.

Die Ergebnisse von Beispiel 2 zeigen, dass ein erheblicher Spezifitätsgewinn von 6% gegenüber dem herkömmlichen Test erzielt werden kann.

Es ist zu betonen, dass bereits der herkömmliche ELISA eine erhebliche Spezifität von 93% zeigt. Eine weitere Erhöhung der Spezifität auf diesem hohen Niveau zu erreichen, ist in der Diagnostik technisch anspruchsvoll und ein erheblicher Beitrag zum Stand der Technik.

Die Spezifität und die diagnostische Zuverlässigkeit insgesamt sind erhöht, wenn das erfindungsgemäße Verfahren zur Anwendung kommt.

### Beispiel 3: Bestimmung der Sensititivät und Spezifität eines Chemilumineszenzbasierten Assays zur Diagnose von AIG mittels Bestimmung von Autoantikörper gegen ATP4B

Soweit nicht anders beschrieben, wurde der Versuch mit den gleichen Reagenzien durchgeführt, wie sie in Beispiel 1 beschrieben sind.

Im vorliegenden ChLIA werden Antigen-beschichtete Magnetpartikel als Festphase für den spezifischen Nachweis von Autoantikörpern der Immunglobulinklasse IgG gegen ATP4B eingesetzt. Die Magnetpartikel wurden unter Verwendung von tosylaktivierten M-280 Dynabeads (Invitrogen, Katalognummer 14203, 14204) gemäß Anleitung des Herstellers hergestellt.

Die Durchführung des Tests erfolgt automatisiert in einem Random Access Analyzer (RA Analyzer 10, product no. YG 0710-0101, EUROIMMUN Medizinische Labordiagnostika AG).. Im ersten Analyseschritt werden beschichtete Magnetpartikel mit verdünnten Patientenproben in Probenpuffer (Katalognummer LL9012, EUROIMMUN Medizinische Labordiagnostika AG) inkubiert. Sind die Proben anti-ATP4B -positiv, binden sich spezifische Antikörper unter diesen Bedingungen an das Antigen.

In einem zweiten Analyseschritt wird Acridiniumester-markiertes Anti-human-IgG (Katalognummer LK0711-G, EUROIMMUN Medizinische Labordiagnostika AG) Konjugat hinzugegeben, das sich an die spezifischen Antikörper bindet.

Anschließend wird der Reaktionsansatz mit Triggerlösungen (Katalognummern LL0713-1 (A) und LL0713-2 (B), EUROIMMUN) versetzt, die eine Chemilumineszenz-Reaktion induzieren.

Das entstehende Lichtsignal wird in relativen Lichteinheiten (RLU) ausgegeben. Die Intensität des Lichtsignals ist proportional zur Menge der gebundenen Antikörper.

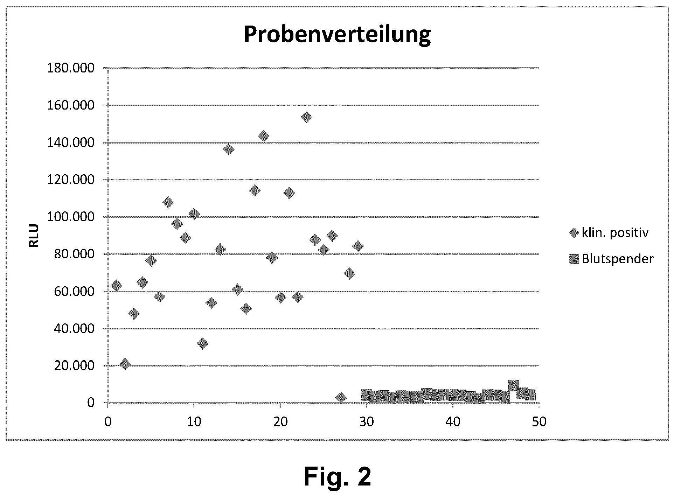

Die Ergebnisse sind in Tabelle 3 gezeigt. Eine Kontrolle mit 20 Seren von Blutspender zeigte eine Spezifität von 100%, mit dem herkömmlichen Assay betrug sie nur 95%. Daten zur Sensitiviät sind in **Tabelle 4** und **Fig. 2** gezeigt. Die Sensitivität betrug bei 29 Patienten, die unter Autoimmungastritis leiden, 96,5% und liegt damit höher als der Wert, den Ma et al. unter Verwendung von nicht aufgereinigter beta-Untereinheit mit ELISA erreichten.

**Tabelle 3: Spezifität des erfindungsgemäßen Assays (Anti-ATP4B-ELISA und Anti-ATP4B-ChLIA) im Vergleich zum herkömmlichen Assay (Anti-PCA-ELISA):**

| | | | Anti-**ATP4B-ELISA** (IgG) (Serum 1:101) | Anti-**ATP4B-ChLIA** (IgG) | Anti-**PCA-ELISA** (IgG) |
|---|---|---|---|---|---|
| | | | Cutoff: 0,284 OD | Cutoff: 15.203 RLU | Cutoff: 20 E/ml |
| | | | normierte OD | RLU | E/ml |
| BS 2014-II-247 | 52 | M | 0,018 | 4.148 | **37** |
| BS 2014-II-248 | 51 | M | 0,011 | 3.146 | < 2 |
| BS 2014-II-249 | 69 | M | 0,007 | 3.821 | 12 |
| BS 2014-II-251 | 31 | M | 0,024 | 2.579 | < 2 |
| BS 2014-II-252 | 51 | M | 0,007 | 3.712 | 15 |
| BS 2014-II-254 | 21 | M | 0,018 | 3.080 | < 2 |
| BS 2014-II-255 | 21 | M | 0,021 | 2.997 | < 2 |
| BS 2014-II-256 | 42 | M | 0,014 | 4.835 | 12 |
| BS 2014-II-257 | 30 | W | 0,021 | 4.098 | < 2 |
| BS 2014-II-259 | 27 | W | 0,016 | 4.306 | 8 |
| BS 2014-II-262 | 44 | M | 0,015 | 4.165 | 2 |
| BS 2014-II-263 | 22 | M | 0,018 | 3.922 | 6 |
| BS 2014-II-264 | 24 | M | 0,017 | 3.305 | < 2 |
| BS 2014-II-266 | 26 | M | 0,015 | 2.285 | < 2 |
| BS 2014-II-268 | 64 | M | 0,014 | 4.419 | < 2 |
| BS 2014-II-269 | 59 | M | 0,021 | 4.057 | 6 |
| BS 2014-II-270 | 20 | W | 0,009 | 2.941 | < 2 |
| BS 2014-II-274 | 21 | M | 0,035 | 9.391 | 2 |
| BS 2014-II-275 | 54 | M | 0,015 | 5.130 | 2 |
| BS 2014-II-276 | 33 | W | 0,032 | 4.446 | 4 |

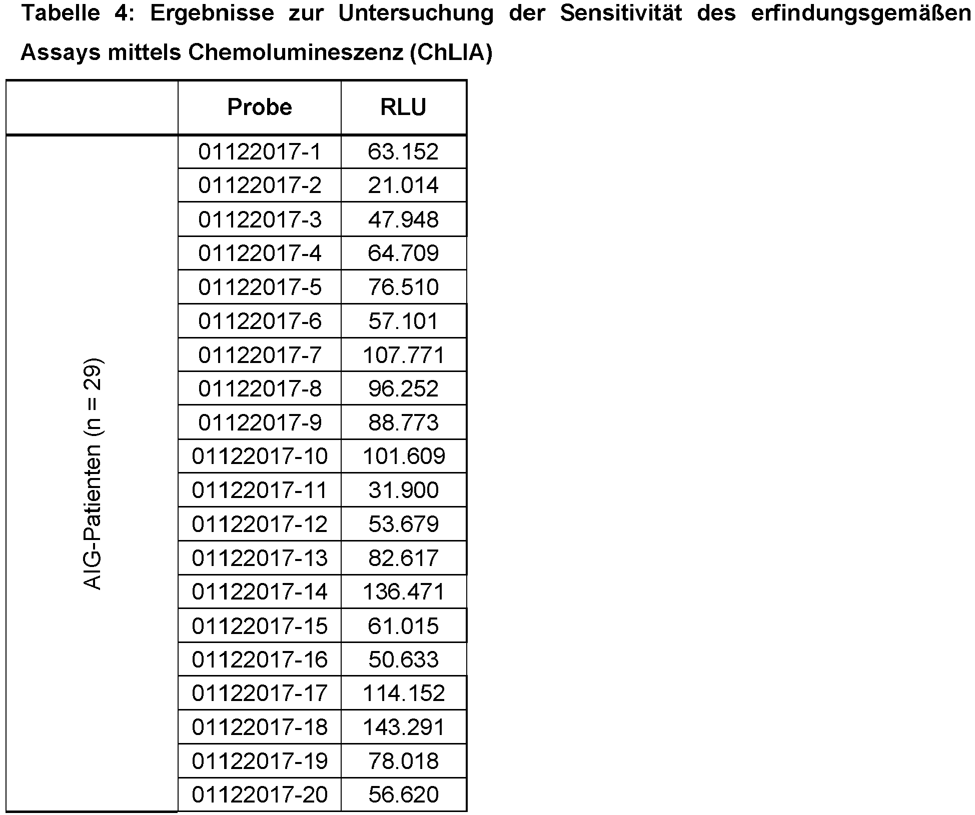

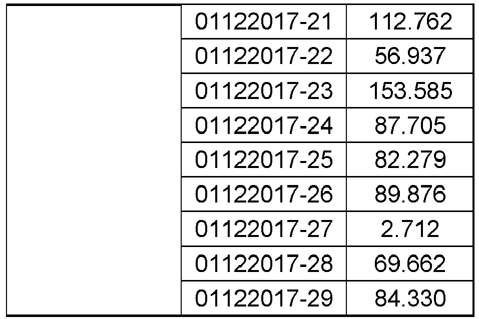

## Patentansprüche

1. Verfahren zur Diagnose von AIG umfassend den Schritt Nachweisen, ob in einer Probe, die Antikörper enthält, Autoantikörper gegen die beta-Untereinheit der gastritischen H⁺/K⁺-ATPase vorhanden oder nicht vorhanden sind, wobei ein diagnostisch nützlicher Träger mit einer Festphase eingesetzt wird, an der ein Mittel zum spezifischen Einfangen des Autoantikörpers immobilisiert ist.

2. Verfahren nach Anspruch 1, wobei der Träger aus der Gruppe ausgewählt ist, die eine Mikrotiterplatte, einen Bead, einen Blot; bevorzugt Western Blot, Lineblot oder Dot Blot; einen Immunfluoreszenztestträger mit fixierten Zellen, bevorzugt für eine mikroskopische Analyse, eine Lateral Flow-Vorrichtung, ein zellulosebasiertes Polymer, einen Biochip, einen Mikroarray, und ein Chromatographiesäulenmaterial umfasst.

3. Verfahren nach Anspruch 2, wobei es sich bei dem diagnostisch nützlichen Träger um eine Mikrotiterplatte für ELISA handelt.

4. Verfahren nach einem der Ansprüche 1 bis 3, wobei die Probe aus der Gruppe ausgewählt ist, die Serum, Vollblut, Plasma, CSF umfasst.

5. Verfahren nach einem der Ansprüche 1 bis 4, weiter umfassend Nachweisen, ob in einer Probe Autoantikörper gegen den Intrinsic Factor vorhanden oder nicht vorhanden sind.

6. Verfahren nach einem der Ansprüche 1 bis 5, wobei der Autoantikörper ein Antikörper der Klasse IgG ist.

7. Diagnostisch nützlicher Träger mit einer Festphase, an der ein Mittel zum spezifischen Einfangen eines Autoantikörpers gegen die beta-Untereinheit der gastritischen H⁺/K⁺-ATPase immobilisiert ist.

8. Träger nach Anspruch 7, ausgewählt aus der Gruppe, die eine Mikrotiterplatte, einen Bead, einen Blot; bevorzugt Western Blot, Lineblot oder Dot Blot; einen Immunfluoreszenztestträger mit fixierten Zellen, bevorzugt für eine mikroskopische Analyse, eine Lateral Flow-Vorrichtung, ein zellulosebasiertes Polymer, einen Biochip, einen Mikroarray, und ein Chromatographiesäulenmaterial umfasst.

9. Träger nach Anspruch 8, wobei es sich um eine Mikrotiterplatte für ELISA handelt.

10. Monoklonaler Antikörper oder isolierter Autoantikörper, der spezifisch gegen die beta-Untereinheit der gastritischen H⁺/K⁺-ATPase bindet, besonders bevorzugt in aufgereinigter Form.

11. Verfahren zum Kalibrieren oder zur Kontrolle der Qualität eines Medizinproduktes, umfassend den Schritt Kontaktieren des Trägers nach einem der Ansprüche 7 bis 9 mit einer Flüssigkeit umfassend einen Antikörper gemäß Anspruch 10, bevorzugt in bekannter Konzentration, gefolgt von dem Schritt Nachweisen, ob der Antikörper in der Flüssigkeit vorhanden oder nicht vorhanden ist, besonders bevorzugt unter wenigstens semi-quantitativer Bestimmung des Antikörpers in der Flüssigkeit.

12. Kit umfassend den diagnostisch nützlichen Träger gemäß einem der Ansprüche 7 bis 9, wobei der Kit ein oder mehr als ein Reagenz, bevorzugt alle Reagenzien, aus der Gruppe enthält, die eine Waschlösung, eine Kalibratorlösung, einen Antikörper gegen die beta-Untereinheit der gastritischen H⁺/K⁺-ATPase, und ein Mittel zum Detektieren des Autoantikörpers, bevorzugt einen sekundären Antikörper, noch bevorzugter einen sekundären Antikörper gegen IgG-Antikörper oder die beta-Untereinheit der gastritischen H⁺/K⁺-ATPase oder eine Variante davon, bevorzugt mit nachweisbarer Markierung, umfasst.

13. Kit nach Anspruch 12, wobei der sekundäre Antikörper eine nachweisbare Markierung aufweist, bevorzugt aus der Gruppe umfassend eine chemilumineszenzfähige, radioaktive oder ein nachweisbares Isotop umfassende Markierung, eine enzymatisch aktive Markierung, eine fluoreszenzfähige Markierung, eine durch NMR-Spektroskopie nachweisbare Verbindung, eine Spin Label-Markierung, bevorzugt eine chemilumineszenzfähige Markierung, am bevorzugtesten eine chemilumineszenzfähige Markierung, die bei Inkubation in einer geeigneten Chemilumineszenz-Triggerlösung ein Chemilumineszenz-Signal erzeugt.

14. Verwendung eines Mittels zum spezifischen Einfangen eines Autoantikörpers gegen die beta-Untereinheit der gastritischen H⁺/K⁺-ATPase oder des Trägers gemäß einem der Ansprüche 7 bis 9 oder des Antikörpers nach Anspruch 10 oder eines Polypeptides umfassend die beta-Untereinheit der gastritischen H⁺/K⁺-ATPase, bevorzugt der extrazellulären Domäne der beta-Untereinheit der gastritischen H⁺/K⁺-ATPase oder einer Variante davon oder einer dafür kodierenden Nukleinsäure zur hochspezifischen Diagnose von AIG.

15. Verwendung eines Mittels zum spezifischen Einfangen eines Autoantikörpers gegen die beta-Untereinheit der gastritischen H⁺/K⁺-ATPase oder eines Trägers umfassend das Mittel oder der beta-Untereinheit der gastritischen H⁺/K⁺-ATPase, bevorzugt der extrazellulären Domäne der beta-Untereinheit der gastritischen H⁺/K⁺-ATPase oder einer Variante davon oder einer dafür kodierenden Nukleinsäure zur Herstellung eines Kits für die hochspezifische Diagnose von AIG.


SEQUENCE LISTING

<110>  EUROIMMUN Medizinische Labordiagnostika AG

<120>  Verfahren zur Diagnose von Autoimmungastritis

<130>  18PP265EP

<160>  12

<170>  PatentIn version 3.5

<210>  1
<211>  258
<212>  PRT
<213>  artificial

<220>
<223>  spGh*-H6-ATP4B(ec)

<400>  1

Met Lys His Leu Trp Phe Phe Leu Leu Leu Val Ala Ala Pro Arg Trp
1               5                   10                  15

Val Leu Ser Gly Pro Met His His His His His His Met Ser Gln Thr
            20                  25                  30

Val Asp Pro Tyr Thr Pro Asp Tyr Gln Asp Gln Leu Arg Ser Pro Gly
        35                  40                  45

Val Thr Leu Arg Pro Asp Val Tyr Gly Glu Lys Gly Leu Glu Ile Val
    50                  55                  60

Tyr Asn Val Ser Asp Asn Arg Thr Trp Ala Asp Leu Thr Gln Thr Leu
65                  70                  75                  80

His Ala Phe Leu Ala Gly Tyr Ser Pro Ala Ala Gln Glu Asp Ser Ile
                85                  90                  95

Asn Cys Thr Ser Glu Gln Tyr Phe Phe Gln Glu Ser Phe Arg Ala Pro
            100                 105                 110

Asn His Thr Lys Phe Ser Cys Lys Phe Thr Ala Asp Met Leu Gln Asn
        115                 120                 125

Cys Ser Gly Leu Ala Asp Pro Asn Phe Gly Phe Glu Glu Gly Lys Pro
    130                 135                 140

Cys Phe Ile Ile Lys Met Asn Arg Ile Val Lys Phe Leu Pro Ser Asn
145                 150                 155                 160

Gly Ser Ala Pro Arg Val Asp Cys Ala Phe Leu Asp Gln Pro Arg Glu

                165                 170                 175

Leu Gly Gln Pro Leu Gln Val Lys Tyr Tyr Pro Pro Asn Gly Thr Phe
            180                 185                 190

Ser Leu His Tyr Phe Pro Tyr Tyr Gly Lys Lys Ala Gln Pro His Tyr
        195                 200                 205

Ser Asn Pro Leu Val Ala Ala Lys Leu Leu Asn Ile Pro Arg Asn Ala
    210                 215                 220

Glu Val Ala Ile Val Cys Lys Val Met Ala Glu His Val Thr Phe Asn
225                 230                 235                 240

Asn Pro His Asp Pro Tyr Glu Gly Lys Val Glu Phe Lys Leu Lys Ile
                245                 250                 255

Glu Lys

<210> 2
<211> 239
<212> PRT
<213> artificial

<220>
<223> spGh*-H6-ATP4B(ec) extrazelluläre Domäne, mit N-terminalem
His-Tag

<400> 2

Gly Pro Met His His His His His His Met Ser Gln Thr Val Asp Pro
1               5                   10                  15

Tyr Thr Pro Asp Tyr Gln Asp Gln Leu Arg Ser Pro Gly Val Thr Leu
            20                  25                  30

Arg Pro Asp Val Tyr Gly Glu Lys Gly Leu Glu Ile Val Tyr Asn Val
        35                  40                  45

Ser Asp Asn Arg Thr Trp Ala Asp Leu Thr Gln Thr Leu His Ala Phe
    50                  55                  60

Leu Ala Gly Tyr Ser Pro Ala Ala Gln Glu Asp Ser Ile Asn Cys Thr
65                  70                  75                  80

Ser Glu Gln Tyr Phe Phe Gln Glu Ser Phe Arg Ala Pro Asn His Thr
                85                  90                  95

Lys Phe Ser Cys Lys Phe Thr Ala Asp Met Leu Gln Asn Cys Ser Gly

100                 105                 110

Leu Ala Asp Pro Asn Phe Gly Phe Glu Glu Gly Lys Pro Cys Phe Ile
        115                 120                 125

Ile Lys Met Asn Arg Ile Val Lys Phe Leu Pro Ser Asn Gly Ser Ala
    130                 135                 140

Pro Arg Val Asp Cys Ala Phe Leu Asp Gln Pro Arg Glu Leu Gly Gln
145                 150                 155                 160

Pro Leu Gln Val Lys Tyr Tyr Pro Pro Asn Gly Thr Phe Ser Leu His
                165                 170                 175

Tyr Phe Pro Tyr Tyr Gly Lys Lys Ala Gln Pro His Tyr Ser Asn Pro
            180                 185                 190

Leu Val Ala Ala Lys Leu Leu Asn Ile Pro Arg Asn Ala Glu Val Ala
        195                 200                 205

Ile Val Cys Lys Val Met Ala Glu His Val Thr Phe Asn Asn Pro His
    210                 215                 220

Asp Pro Tyr Glu Gly Lys Val Glu Phe Lys Leu Lys Ile Glu Lys
225                 230                 235

<210>  3
<211>  228
<212>  PRT
<213>  artificial

<220>
<223>  Extrazelluläre Domäne der ATP4B-Untereinheit

<400>  3

Gln Thr Val Asp Pro Tyr Thr Pro Asp Tyr Gln Asp Gln Leu Arg Ser
1               5                   10                  15

Pro Gly Val Thr Leu Arg Pro Asp Val Tyr Gly Glu Lys Gly Leu Glu
            20                  25                  30

Ile Val Tyr Asn Val Ser Asp Asn Arg Thr Trp Ala Asp Leu Thr Gln
        35                  40                  45

Thr Leu His Ala Phe Leu Ala Gly Tyr Ser Pro Ala Ala Gln Glu Asp
    50                  55                  60

Ser Ile Asn Cys Thr Ser Glu Gln Tyr Phe Phe Gln Glu Ser Phe Arg
65                  70                  75                  80

Ala Pro Asn His Thr Lys Phe Ser Cys Lys Phe Thr Ala Asp Met Leu
                85                  90                  95

Gln Asn Cys Ser Gly Leu Ala Asp Pro Asn Phe Gly Phe Glu Glu Gly
            100                 105                 110

Lys Pro Cys Phe Ile Ile Lys Met Asn Arg Ile Val Lys Phe Leu Pro
        115                 120                 125

Ser Asn Gly Ser Ala Pro Arg Val Asp Cys Ala Phe Leu Asp Gln Pro
    130                 135                 140

Arg Glu Leu Gly Gln Pro Leu Gln Val Lys Tyr Tyr Pro Pro Asn Gly
145                 150                 155                 160

Thr Phe Ser Leu His Tyr Phe Pro Tyr Tyr Gly Lys Lys Ala Gln Pro
                165                 170                 175

His Tyr Ser Asn Pro Leu Val Ala Ala Lys Leu Leu Asn Ile Pro Arg
            180                 185                 190

Asn Ala Glu Val Ala Ile Val Cys Lys Val Met Ala Glu His Val Thr
        195                 200                 205

Phe Asn Asn Pro His Asp Pro Tyr Glu Gly Lys Val Glu Phe Lys Leu
    210                 215                 220

Lys Ile Glu Lys
225


<210>  4
<211>  23
<212>  PRT
<213>  artificial

<220>
<223>  Transmembrandomäne der ATP4B-Untereinheit

<400>  4

Ser Leu Tyr Tyr Val Ala Phe Tyr Val Val Met Thr Gly Leu Phe Ala
1               5                   10                  15

Leu Cys Leu Tyr Val Leu Met
            20


<210>  5
<211>  25
<212>  PRT

<213>  artificial

<220>
<223>  Immunodominantes Epitop der humanen ATP4B-Untereinheit

<400>  5

Leu Leu Asn Ile Pro Arg Asn Ala Glu Val Ala Ile Val Cys Lys Val
1               5                   10                  15

Met Ala Glu His Val Thr Phe Asn Asn
            20                  25

<210>  6
<211>  25
<212>  PRT
<213>  artificial

<220>
<223>  Immunodominantes Epitop der murinen ATP4B-Untereinheit

<400>  6

Leu Leu Asn Val Pro Lys Asn Met Gln Val Ser Ile Val Cys Lys Ile
1               5                   10                  15

Leu Ala Asp His Val Thr Phe Asn Asn
            20                  25

<210>  7
<211>  291
<212>  PRT
<213>  artificial

<220>
<223>  Aminosäuresequenz der ATP4B-Untereinheit (P51164)

<400>  7

Met Ala Ala Leu Gln Glu Lys Lys Thr Cys Gly Gln Arg Met Glu Glu
1               5                   10                  15

Phe Gln Arg Tyr Cys Trp Asn Pro Asp Thr Gly Gln Met Leu Gly Arg
            20                  25                  30

Thr Leu Ser Arg Trp Val Trp Ile Ser Leu Tyr Tyr Val Ala Phe Tyr
        35                  40                  45

Val Val Met Thr Gly Leu Phe Ala Leu Cys Leu Tyr Val Leu Met Gln
    50                  55                  60

Thr Val Asp Pro Tyr Thr Pro Asp Tyr Gln Asp Gln Leu Arg Ser Pro
65                  70                  75                  80

Gly Val Thr Leu Arg Pro Asp Val Tyr Gly Glu Lys Gly Leu Glu Ile
                85                  90                  95

Val Tyr Asn Val Ser Asp Asn Arg Thr Trp Ala Asp Leu Thr Gln Thr
            100                 105                 110

Leu His Ala Phe Leu Ala Gly Tyr Ser Pro Ala Ala Gln Glu Asp Ser
        115                 120                 125

Ile Asn Cys Thr Ser Glu Gln Tyr Phe Phe Gln Glu Ser Phe Arg Ala
    130                 135                 140

Pro Asn His Thr Lys Phe Ser Cys Lys Phe Thr Ala Asp Met Leu Gln
145                 150                 155                 160

Asn Cys Ser Gly Leu Ala Asp Pro Asn Phe Gly Phe Glu Glu Gly Lys
                165                 170                 175

Pro Cys Phe Ile Ile Lys Met Asn Arg Ile Val Lys Phe Leu Pro Ser
            180                 185                 190

Asn Gly Ser Ala Pro Arg Val Asp Cys Ala Phe Leu Asp Gln Pro Arg
        195                 200                 205

Glu Leu Gly Gln Pro Leu Gln Val Lys Tyr Tyr Pro Pro Asn Gly Thr
    210                 215                 220

Phe Ser Leu His Tyr Phe Pro Tyr Tyr Gly Lys Lys Ala Gln Pro His
225                 230                 235                 240

Tyr Ser Asn Pro Leu Val Ala Ala Lys Leu Leu Asn Ile Pro Arg Asn
                245                 250                 255

Ala Glu Val Ala Ile Val Cys Lys Val Met Ala Glu His Val Thr Phe
            260                 265                 270

Asn Asn Pro His Asp Pro Tyr Glu Gly Lys Val Glu Phe Lys Leu Lys
        275                 280                 285

Ile Glu Lys
    290

<210>  8
<211>  19
<212>  PRT
<213>  artificial

<220>
<223>  Immunodominantes Epitop der murinen ATP4A-Untereinheit

<400>  8

Pro Leu Leu Cys Val Gly Leu Arg Ala Gln Trp Glu Asp His His Leu
1               5                   10                  15

Gln Asp Leu

<210>  9
<211>  1035
<212>  PRT
<213>  artificial

<220>
<223>  alpha-Untereinheit der gastritischen H+/K+-ATPase (P20648)

<400>  9

Met Gly Lys Ala Glu Asn Tyr Glu Leu Tyr Ser Val Glu Leu Gly Pro
1               5                   10                  15

Gly Pro Gly Gly Asp Met Ala Ala Lys Met Ser Lys Lys Lys Lys Ala
            20                  25                  30

Gly Gly Gly Gly Gly Lys Arg Lys Glu Lys Leu Glu Asn Met Lys Lys
        35                  40                  45

Glu Met Glu Ile Asn Asp His Gln Leu Ser Val Ala Glu Leu Glu Gln
    50                  55                  60

Lys Tyr Gln Thr Ser Ala Thr Lys Gly Leu Ser Ala Ser Leu Ala Ala
65                  70                  75                  80

Glu Leu Leu Leu Arg Asp Gly Pro Asn Ala Leu Arg Pro Pro Arg Gly
                85                  90                  95

Thr Pro Glu Tyr Val Lys Phe Ala Arg Gln Leu Ala Gly Gly Leu Gln
            100                 105                 110

Cys Leu Met Trp Val Ala Ala Ala Ile Cys Leu Ile Ala Phe Ala Ile
        115                 120                 125

Gln Ala Ser Glu Gly Asp Leu Thr Thr Asp Asp Asn Leu Tyr Leu Ala
    130                 135                 140

Ile Ala Leu Ile Ala Val Val Val Val Thr Gly Cys Phe Gly Tyr Tyr
145                 150                 155                 160

Gln Glu Phe Lys Ser Thr Asn Ile Ile Ala Ser Phe Lys Asn Leu Val
                165                 170                 175

Pro Gln Gln Ala Thr Val Ile Arg Asp Gly Asp Lys Phe Gln Ile Asn
            180                 185                 190

Ala Asp Gln Leu Val Val Gly Asp Leu Val Glu Met Lys Gly Gly Asp
        195                 200                 205

Arg Val Pro Ala Asp Ile Arg Ile Leu Ala Ala Gln Gly Cys Lys Val
    210                 215                 220

Asp Asn Ser Ser Leu Thr Gly Glu Ser Glu Pro Gln Thr Arg Ser Pro
225                 230                 235                 240

Glu Cys Thr His Glu Ser Pro Leu Glu Thr Arg Asn Ile Ala Phe Phe
                245                 250                 255

Ser Thr Met Cys Leu Glu Gly Thr Val Gln Gly Leu Val Val Asn Thr
            260                 265                 270

Gly Asp Arg Thr Ile Ile Gly Arg Ile Ala Ser Leu Ala Ser Gly Val
        275                 280                 285

Glu Asn Glu Lys Thr Pro Ile Ala Ile Glu Ile Glu His Phe Val Asp
    290                 295                 300

Ile Ile Ala Gly Leu Ala Ile Leu Phe Gly Ala Thr Phe Phe Ile Val
305                 310                 315                 320

Ala Met Cys Ile Gly Tyr Thr Phe Leu Arg Ala Met Val Phe Phe Met
                325                 330                 335

Ala Ile Val Val Ala Tyr Val Pro Glu Gly Leu Leu Ala Thr Val Thr
            340                 345                 350

Val Cys Leu Ser Leu Thr Ala Lys Arg Leu Ala Ser Lys Asn Cys Val
        355                 360                 365

Val Lys Asn Leu Glu Ala Val Glu Thr Leu Gly Ser Thr Ser Val Ile
    370                 375                 380

Cys Ser Asp Lys Thr Gly Thr Leu Thr Gln Asn Arg Met Thr Val Ser
385                 390                 395                 400

His Leu Trp Phe Asp Asn His Ile His Thr Ala Asp Thr Thr Glu Asp
                405                 410                 415

Gln Ser Gly Gln Thr Phe Asp Gln Ser Ser Glu Thr Trp Arg Ala Leu

            420                 425                 430

Cys Arg Val Leu Thr Leu Cys Asn Arg Ala Ala Phe Lys Ser Gly Gln
        435                 440                 445

Asp Ala Val Pro Val Pro Lys Arg Ile Val Ile Gly Asp Ala Ser Glu
    450                 455                 460

Thr Ala Leu Leu Lys Phe Ser Glu Leu Thr Leu Gly Asn Ala Met Gly
465                 470                 475                 480

Tyr Arg Asp Arg Phe Pro Lys Val Cys Glu Ile Pro Phe Asn Ser Thr
                485                 490                 495

Asn Lys Phe Gln Leu Ser Ile His Thr Leu Glu Asp Pro Arg Asp Pro
            500                 505                 510

Arg His Leu Leu Val Met Lys Gly Ala Pro Glu Arg Val Leu Glu Arg
        515                 520                 525

Cys Ser Ser Ile Leu Ile Lys Gly Gln Glu Leu Pro Leu Asp Glu Gln
    530                 535                 540

Trp Arg Glu Ala Phe Gln Thr Ala Tyr Leu Ser Leu Gly Gly Leu Gly
545                 550                 555                 560

Glu Arg Val Leu Gly Phe Cys Gln Leu Tyr Leu Asn Glu Lys Asp Tyr
                565                 570                 575

Pro Pro Gly Tyr Ala Phe Asp Val Glu Ala Met Asn Phe Pro Ser Ser
            580                 585                 590

Gly Leu Cys Phe Ala Gly Leu Val Ser Met Ile Asp Pro Pro Arg Ala
        595                 600                 605

Thr Val Pro Asp Ala Val Leu Lys Cys Arg Thr Ala Gly Ile Arg Val
    610                 615                 620

Ile Met Val Thr Gly Asp His Pro Ile Thr Ala Lys Ala Ile Ala Ala
625                 630                 635                 640

Ser Val Gly Ile Ile Ser Glu Gly Ser Glu Thr Val Glu Asp Ile Ala
                645                 650                 655

Ala Arg Leu Arg Val Pro Val Asp Gln Val Asn Arg Lys Asp Ala Arg
            660                 665                 670

Ala Cys Val Ile Asn Gly Met Gln Leu Lys Asp Met Asp Pro Ser Glu
        675                 680                 685

Leu Val Glu Ala Leu Arg Thr His Pro Glu Met Val Phe Ala Arg Thr
    690                 695                 700

Ser Pro Gln Gln Lys Leu Val Ile Val Glu Ser Cys Gln Arg Leu Gly
705                 710                 715                 720

Ala Ile Val Ala Val Thr Gly Asp Gly Val Asn Asp Ser Pro Ala Leu
                725                 730                 735

Lys Lys Ala Asp Ile Gly Val Ala Met Gly Ile Ala Gly Ser Asp Ala
            740                 745                 750

Ala Lys Asn Ala Ala Asp Met Ile Leu Leu Asp Asp Asn Phe Ala Ser
        755                 760                 765

Ile Val Thr Gly Val Glu Gln Gly Arg Leu Ile Phe Asp Asn Leu Lys
    770                 775                 780

Lys Ser Ile Ala Tyr Thr Leu Thr Lys Asn Ile Pro Glu Leu Thr Pro
785                 790                 795                 800

Tyr Leu Ile Tyr Ile Thr Val Ser Val Pro Leu Pro Leu Gly Cys Ile
                805                 810                 815

Thr Ile Leu Phe Ile Glu Leu Cys Thr Asp Ile Phe Pro Ser Val Ser
            820                 825                 830

Leu Ala Tyr Glu Lys Ala Glu Ser Asp Ile Met His Leu Arg Pro Arg
        835                 840                 845

Asn Pro Lys Arg Asp Arg Leu Val Asn Glu Pro Leu Ala Ala Tyr Ser
    850                 855                 860

Tyr Phe Gln Ile Gly Ala Ile Gln Ser Phe Ala Gly Phe Thr Asp Tyr
865                 870                 875                 880

Phe Thr Ala Met Ala Gln Glu Gly Trp Phe Pro Leu Leu Cys Val Gly
                885                 890                 895

Leu Arg Ala Gln Trp Glu Asp His His Leu Gln Asp Leu Gln Asp Ser
            900                 905                 910

Tyr Gly Gln Glu Trp Thr Phe Gly Gln Arg Leu Tyr Gln Gln Tyr Thr
        915                 920                 925

Cys Tyr Thr Val Phe Phe Ile Ser Ile Glu Val Cys Gln Ile Ala Asp
    930                 935                 940

Val Leu Ile Arg Lys Thr Arg Arg Leu Ser Ala Phe Gln Gln Gly Phe
945                 950                 955                 960

Phe Arg Asn Lys Ile Leu Val Ile Ala Ile Val Phe Gln Val Cys Ile
                965                 970                 975

Gly Cys Phe Leu Cys Tyr Cys Pro Gly Met Pro Asn Ile Phe Asn Phe
            980                 985                 990

Met Pro Ile Arg Phe Gln Trp Trp  Leu Val Pro Leu Pro  Tyr Gly Ile
        995                 1000                 1005

Leu Ile  Phe Val Tyr Asp Glu  Ile Arg Lys Leu Gly  Val Arg Cys
    1010                 1015                 1020

Cys Pro  Gly Ser Trp Trp Asp  Gln Glu Leu Tyr Tyr
    1025                 1030                 1035

<210>  10
<211>  417
<212>  PRT
<213>  artificial

<220>
<223>  Intrinsic factor (NP_005133.2)

<400>  10

Met Ala Trp Phe Ala Leu Tyr Leu Leu Ser Leu Leu Trp Ala Thr Ala
1               5                   10                  15

Gly Thr Ser Thr Gln Thr Gln Ser Ser Cys Ser Val Pro Ser Ala Gln
            20                  25                  30

Glu Pro Leu Val Asn Gly Ile Gln Val Leu Met Glu Asn Ser Val Thr
        35                  40                  45

Ser Ser Ala Tyr Pro Asn Pro Ser Ile Leu Ile Ala Met Asn Leu Ala
    50                  55                  60

Gly Ala Tyr Asn Leu Lys Ala Gln Lys Leu Leu Thr Tyr Gln Leu Met
65                  70                  75                  80

Ser Ser Asp Asn Asn Asp Leu Thr Ile Gly Gln Leu Gly Leu Thr Ile
                85                  90                  95

Met Ala Leu Thr Ser Ser Cys Arg Asp Pro Gly Asp Lys Val Ser Ile
        100                 105                 110

Leu Gln Arg Gln Met Glu Asn Trp Ala Pro Ser Ser Pro Asn Ala Glu
    115                 120                 125

Ala Ser Ala Phe Tyr Gly Pro Ser Leu Ala Ile Leu Ala Leu Cys Gln
    130                 135                 140

Lys Asn Ser Glu Ala Thr Leu Pro Ile Ala Val Arg Phe Ala Lys Thr
145                 150                 155                 160

Leu Leu Ala Asn Ser Ser Pro Phe Asn Val Asp Thr Gly Ala Met Ala
                165                 170                 175

Thr Leu Ala Leu Thr Cys Met Tyr Asn Lys Ile Pro Val Gly Ser Glu
            180                 185                 190

Glu Gly Tyr Arg Ser Leu Phe Gly Gln Val Leu Lys Asp Ile Val Glu
        195                 200                 205

Lys Ile Ser Met Lys Ile Lys Asp Asn Gly Ile Ile Gly Asp Ile Tyr
    210                 215                 220

Ser Thr Gly Leu Ala Met Gln Ala Leu Ser Val Thr Pro Glu Pro Ser
225                 230                 235                 240

Lys Lys Glu Trp Asn Cys Lys Lys Thr Thr Asp Met Ile Leu Asn Glu
                245                 250                 255

Ile Lys Gln Gly Lys Phe His Asn Pro Met Ser Ala Ile Gln Ile Leu
            260                 265                 270

Pro Ser Leu Lys Gly Lys Thr Tyr Leu Asp Val Pro Gln Val Thr Cys
        275                 280                 285

Ser Pro Asp His Glu Val Gln Pro Thr Leu Pro Ser Asn Pro Gly Pro
    290                 295                 300

Gly Pro Thr Ser Ala Ser Asn Ile Thr Val Ile Tyr Thr Ile Asn Asn
305                 310                 315                 320

Gln Leu Arg Gly Val Glu Leu Leu Phe Asn Glu Thr Ile Asn Val Ser
                325                 330                 335

Val Lys Ser Gly Ser Val Leu Leu Val Val Leu Glu Glu Ala Gln Arg
            340                 345                 350

Lys Asn Pro Met Phe Lys Phe Glu Thr Thr Met Thr Ser Trp Gly Leu
        355                 360                 365

Val Val Ser Ser Ile Asn Asn Ile Ala Glu Asn Val Asn His Lys Thr
    370                 375                 380

Tyr Trp Gln Phe Leu Ser Gly Val Thr Pro Leu Asn Glu Gly Val Ala
385                 390                 395                 400

Asp Tyr Ile Pro Phe Asn His Glu His Ile Thr Ala Asn Phe Thr Gln
                405                 410                 415

Tyr

<210>  11
<211>  8752
<212>  DNA
<213>  artificial

<220>
<223>  cDNA kodierend für die ATP4A-Untereinheit ligiert in den Vector
       pTriEx

<400>  11
   ggggaattgt gagcggataa caattccccg gagttaatcc gggaccttta attcaaccca      60
   acacaatata ttatagttaa ataagaatta ttatcaaatc atttgtatat taattaaaat     120
   actatactgt aaattacatt ttatttacaa tcaaaggaga tataggccgt caaggcccac     180
   catggggaag gccgagaact atgagctcta ctcggtggag ctgggtcctg gccctggcgg     240
   ggacatggct gccaagatga gcaagaagaa gaaggcgggt ggcgggggtg gcaagaggaa     300
   ggagaagctg gagaacatga agaaggagat ggagattaac gaccaccagc tgtcagtggc     360
   ggagctggaa cagaaatacc agaccagtgc caccaagggc ctctctgcga gcctggctgc     420
   tgagctgctg ctgcgggatg ggcccaacgc actgcggcca ccacggggca ccccagagta     480
   cgtcaagttc gcgaggcagc tggccggggg cctgcagtgc ctcatgtggg ttgccgccgc     540
   catctgcctc atcgcctttg ccatccaggc tagtgagggg gacctcacca ccgacgacaa     600
   tctgtacctg gcaatcgctc tcattgctgt ggttgtcgtc accggctgct ttggctacta     660
   ccaggaattc aagagcacca acatcatcgc cagctttaag aaccttgtgc cacagcaagc     720
   cactgtcatc cgcgatggag acaaattcca gatcaacgct gaccaactgg tggtgggcga     780
   cctggtggag atgaaaggtg gggacagagt gcccgccgac atccgcatcc tggcggccca     840
   gggctgcaag gtggacaact cctcgctgac aggggagtct gagccacaga cccgctcacc     900
   cgagtgcacg cacgagagcc ctctggagac ccgcaacatc gccttcttct ccaccatgtg     960
   ccttgagggc accgtgcagg gcctggtggt gaacacgggc gaccgcacca tcattgggcg 1020
   catcgcatcg ctggcgtcgg gggtggaaaa cgagaagaca cccatcgcta tcgagatcga 1080
   gcattttgtg gacatcatcg cgggcctggc cattctcttc ggtgccacat tttttattgt 1140
   ggccatgtgc attggctaca ccttcctgcg ggccatggtc ttcttcatgg ccatcgtggt 1200
   ggcctatgtg cctgaggggc tgctggccac tgtcacagtc tgcctgtccc tgacagccaa 1260
   gcgcctggcc agtaagaact gcgtggtcaa gaacctggag gcggtggaga cattgggctc 1320
   cacttcggtg atctgctcgg acaagacagg gactctcact cagaaccgca tgactgtgtc 1380
   ccatctgtgg tttgacaacc acatccacac agctgacacc acggaagacc agtcagggca 1440
   gacgtttgac cagtcctcgg agacgtggcg ggcgctgtgc cgggtgctca ccctgtgcaa 1500
   ccgcgccgcc ttcaagtccg gccaggatgc agtgcctgtg cccaagcgca tcgtgattgg 1560
   agacgcatcg gagacggcgc tgctcaagtt ctcggagctg acgctgggca acgccatggg 1620
   ctaccgggac cgcttcccaa aagtctgcga gatacccttc aactccacca acaagttcca 1680
   gctgtccatc catacgctgg aggacccgcg ggacccgcga cacttgctgg tgatgaaggg 1740
   cgcccccgag cgcgtgctgg agcgctgcag ctccatcctt atcaagggcc aggagctgcc 1800
   gctggacgag cagtggcgcg aggccttcca gaccgcctac ctcagcctgg gaggcctggg 1860
   cgaacgcgtg ctcggcttct gccagctcta cctgaatgag aaggactacc cgcctggcta 1920
   tgccttcgac gtagaggcca tgaactttcc atctagcggc ctctgctttg cgggacttgt 1980
   atccatgatt gacccacccc gggccaccgt ccctgatgct gtgctcaagt gtcgcaccgc 2040
   aggcatccgg gtgatcatgg taacgggtga ccaccccatc accgccaagg ccattgcagc 2100
   cagtgtgggc atcatctcgg aaggcagcga gacagtggag gacatcgctg cccgcctccg 2160
   tgtgcccgta gaccaggtta atcgcaagga tgcccgtgcc tgtgtgatca atggcatgca 2220
   gctgaaggac atggacccat cggaactggt cgaggccctg cgcacccacc ccgagatggt 2280
   gtttgcgcgc accagccccc agcagaagct ggtgatcgtg gagagctgcc agcggctggg 2340
   tgcgattgtg gccgtcacgg gggatggtgt gaatgactcc ccagctctga agaaggcaga 2400
   catcggagta gccatgggca tcgctggctc agatgctgcc aaaaatgcag ctgacatgat 2460
   cctgctggat gacaactttg cctccattgt gacaggcgtg gagcagggtc gactgatctt 2520
   cgacaacctg aagaagtcta ttgcctacac attgaccaag aacatcccag agctgacacc 2580
   ctacctcatc tacatcaccg tcagcgtgcc cctgcccctc gggtgcatca ccatcctctt 2640
   catcgaactc tgcactgaca ttttcccatc tgtgtccctg gcatatgaaa aggccgagag 2700
   tgacatcatg cacctgcgtc cacgcaaccc aaagcgtgac agattggtca acgagcccct 2760
   ggctgcctac tcctacttcc agattggtgc cattcagtcc tttgctggct tcactgacta 2820
   cttcacggca atggcccagg agggctggtt cccactgctg tgcgtggggc tgcgggcgca 2880
   gtgggaggac caccacctac aagatctgca ggacagctac ggccaggagt ggacattcgg 2940
   gcagcgcctg taccagcagt acacctgcta caccgtgttc ttcatcagca ttgaggtgtg 3000
   ccagatcgcc gatgtcctca tccgcaagac gcgccgtctc tctgccttcc agcaaggctt 3060
   cttcaggaat aagatcctgg tgatcgccat cgtgttccag gtctgcatcg gctgcttcct 3120
   gtgctactgc cccggcatgc ccaacatctt caacttcatg cccattcggt tccagtggtg 3180
   gctggtcccc ctgccctacg gcatcctcat cttcgtctat gatgagatcc ggaagcttgg 3240
   agttcgctgt tgcccaggga gctggtggga ccaggaactc tactattagg gcctcatggg 3300
   ccggcgcgcc caccaccacc accaccactg acactaagtg attaacctca ggtgcaggct 3360
   gcctatcaga aggtggtggc tggtgtggcc aatgccctgg ctcacaaata ccactgagat 3420
   cgatcttttt ccctctgcca aaaattatgg ggacatcatg aagccccttg agcatctgac 3480
   ttctggctaa taaaggaaat ttattttcat tgcaatagtg tgttggaatt ttttgtgtct 3540
   ctcactcgga aggacatatg ggagggcaaa tcatttaaaa catcagaatg agtatttggt 3600
   ttagagtttg gcaacatatg cccatatgta actagcataa ccccttgggg cctctaaacg 3660
   ggtcttgagg ggttttttgc tgaaagcatg cggaggaaat tctccttgaa gtttccctgg 3720
   tgttcaaagt aaaggagttt gcaccagacg cacctctgtt cactggtccg gcgtattaaa 3780
   acacgataca ttgttattag tacatttatt aagcgctaga ttctgtgcgt tgttgattta 3840
   cagacaattg ttgtacgtat tttaataatt cattaaattt ataatcttta gggtggtatg 3900
   ttagagcgaa aatcaaatga ttttcagcgt ctttatatct gaatttaaat attaaatcct 3960
   caatagattt gtaaaatagg tttcgattag tttcaaacaa gggttgtttt tccgaaccga 4020
   tggctggact atctaatgga ttttcgctca acgccacaaa acttgccaaa tcttgtagca 4080
   gcaatctagc tttgtcgata ttcgtttgtg ttttgttttg taataaaggt tcgacgtcgt 4140
   tcaaaatatt atgcgctttt gtatttcttt catcactgtc gttagtgtac aattgactcg 4200
   acgtaaacac gttaaataga gcttggacat atttaacatc gggcgtgtta gctttattag 4260
   gccgattatc gtcgtcgtcc caaccctcgt cgttagaagt tgcttccgaa gacgattttg 4320
   ccatagccac acgacgccta ttaattgtgt cggctaacac gtccgcgatc aaatttgtag 4380
   ttgagctttt tggaattatt tctgattgcg ggcgtttttg ggcgggtttc aatctaactg 4440
   tgcccgattt taattcagac aacacgttag aaagcgatgg tgcaggcggt ggtaacattt 4500
   cagacggcaa atctactaat ggcggcggtg gtggagctga tgataaatct accatcggtg 4560
   gaggcgcagg cggggctggc ggcggaggcg gaggcggagg tggtggcggt gatgcagacg 4620
   gcggtttagg ctcaaatgtc tctttaggca acacagtcgg cacctcaact attgtactgg 4680
   tttcgggcgc cgtttttggt ttgaccggtc tgagacgagt gcgatttttt tcgtttctaa 4740
   tagcttccaa caattgttgt ctgtcgtcta aaggtgcagc gggttgaggt tccgtcggca 4800
   ttggtggagc gggcggcaat tcagacatcg atggtggtgg tggtggtgga ggcgctggaa 4860
   tgttaggcac gggagaaggt ggtggcggcg gtgccgccgg tataatttgt tctggtttag 4920
   tttgttcgcg cacgattgtg ggcaccggcg caggcgccgc tggctgcaca acggaaggtc 4980
   gtctgcttcg aggcagcgct tggggtggtg gcaattcaat attataattg gaatacaaat 5040
   cgtaaaaatc tgctataagc attgtaattt cgctatcgtt taccgtgccg atatttaaca 5100
   accgctcaat gtaagcaatt gtattgtaaa gagattgtct caagctcgga acgctgcgct 5160
   cggtcgttcg gctgcggcga gcggtatcag ctcactcaaa ggcggtaata cggttatcca 5220
   cagaatcagg ggataacgca ggaaagaaca tgtgagcaaa aggccagcaa aaggccagga 5280
   accgtaaaaa ggccgcgttg ctggcgtttt tccataggct ccgcccccct gacgagcatc 5340
   acaaaaatcg acgctcaagt cagaggtggc gaaacccgac aggactataa agataccagg 5400
   cgtttccccc tggaagctcc ctcgtgcgct ctcctgttcc gaccctgccg cttaccggat 5460
   acctgtccgc ctttctccct tcgggaagcg tggcgctttc tcaatgctca cgctgtaggt 5520
   atctcagttc ggtgtaggtc gttcgctcca agctgggctg tgtgcacgaa ccccccgttc 5580
   agcccgaccg ctgcgcctta tccggtaact atcgtcttga gtccaacccg gtaagacacg 5640
   acttatcgcc actggcagca gccactggta acaggattag cagagcgagg tatgtaggcg 5700
   gtgctacaga gttcttgaag tggtggccta actacggcta cactagaagg acagtatttg 5760
   gtatctgcgc tctgctgaag ccagttacct tcggaaaaag agttggtagc tcttgatccg 5820
   gcaaacaaac caccgctggt agcggtggtt tttttgtttg caagcagcag attacgcgca 5880
   gaaaaaaagg atctcaagaa gatcctttgt taccaatgct taatcagtga ggcacctatc 5940
   tcagcgatct gtctatttcg ttcatccata gttgcctgac tccccgtcgt gtagataact 6000
   acgatacggg agggcttacc atctggcccc agtgctgcaa tgataccgcg agacccacgc 6060
   tcaccggctc cagatttatc agcaataaac cagccagccg gaagggccga gcgcagaagt 6120
   ggtcctgcaa ctttatccgc ctccatccag tctattaatt gttgccggga agctagagta 6180
   agtagttcgc cagttaatag tttgcgcaac gttgttgcca ttgctacagg catcgtggtg 6240
   tcacgctcgt cgtttggtat ggcttcattc agctccggtt cccaacgatc aaggcgagtt 6300
   acatgatccc ccatgttgtg caaaaaagcg gttagctcct tcggtcctcc gatcgttgtc 6360
   agaagtaagt tggccgcagt gttatcactc atggttatgg cagcactgca taattctctt 6420
   actgtcatgc catccgtaag atgcttttct gtgactggtg agtactcaac caagtcattc 6480
   tgagaatagt gtatgcggcg accgagttgc tcttgcccgg cgtcaatacg ggataatacc 6540
   gcgccacata gcagaacttt aaaagtgctc atcattggaa aacgttcttc ggggcgaaaa 6600
   ctctcaagga tcttaccgct gttgagatcc agttcgatgt aacccactcg tgcacccaac 6660
   tgatcttcag catcttttac tttcaccagc gtttctgggt gagcaaaaac aggaaggcaa 6720
   aatgccgcaa aaaagggaat aagggcgaca cggaaatgtt gaatactcat actcttcctt 6780
   tttcaatatt attgaagcat ttatcagggt tattgtctca tgtccgcgcg tttcctgcat 6840
   cttttaatca aatcccaaga tgtgtataaa ccaccaaact gccaaaaaat gaaaactgtc 6900
   gacaagctct gtccgtttgc tggcaactgc aagggtctca atcctatttg taattattga 6960
   ataataaaac aattataaat gtcaaatttg ttttttatta acgatacaaa ccaaacgcaa 7020
   caagaacatt tgtagtatta tctataattg aaaacgcgta gttataatcg ctgaggtaat 7080
   atttaaaatc attttcaaat gattcacagt taatttgcga caatataatt ttattttcac 7140
   ataaactaga cgccttgtcg tcttcttctt cgtattcctt ctctttttca tttttctctt 7200
   cataaaaatt aacatagtta ttatcgtatc catatatgta tctatcgtat agagtaaatt 7260
   ttttgttgtc ataaatatat atgtcttttt taatggggtg tatagtaccg ctgcgcatag 7320
   tttttctgta atttacaaca gtgctatttt ctggtagttc ttcggagtgt gttgctttaa 7380
   ttattaaatt tatataatca atgaatttgg gatcgtcggt tttgtacaat atgttgccgg 7440
   catagtacgc agcttcttct agttcaatta caccattttt tagcagcacc ggattaacat 7500
   aactttccaa aatgttgtac gaaccgttaa acaaaaacag ttcacctccc ttttctatac 7560
   tattgtctgc gagcagttgt ttgttgttaa aaataacagc cattgtaatg agacgcacaa 7620
   actaatatca caaactggaa atgtctatca atatatagtt gctctagtta ttaatagtaa 7680
   tcaattacgg ggtcattagt tcatagccca tatatggagt tccgcgttac ataacttacg 7740
   gtaaatggcc cgcctggctg accgcccaac gacccccgcc cattgacgtc aataatgacg 7800
   tatgttccca tagtaacgcc aatagggact ttccattgac gtcaatgggt ggactattta 7860
   cggtaaactg cccacttggc agtacatcaa gtgtatcata tgccaagtac gccccctatt 7920
   gacgtcaatg acggtaaatg gcccgcctgg cattatgccc agtacatgac cttatgggac 7980
   tttcctactt ggcagtacat ctacgtatta gtcatcgcta ttaccatgca tggtcgaggt 8040
   gagccccacg ttctgcttca ctctccccat ctcccccccc tccccacccc caattttgta 8100
   tttatttatt ttttaattat tttgtgcagc gatgggggcg gggggggggg gggggcgcgc 8160
   gccaggcggg gcggggcggg gcgaggggcg gggcggggcg aggcggagag gtgcggcggc 8220
   agccaatcag agcggcgcgc tccgaaagtt tccttttatg gcgaggcggc ggcggcggcg 8280
   gccctataaa aagcgaagcg cgcggcgggc gggagtcgct gcgacgctgc cttcgccccg 8340
   tgccccgctc cgccgccgcc tcgcgccgcc cgccccggct ctgactgacc gcgttactcc 8400
   cacaggtgag cgggcgggac ggcccttctc cttcgggctg taattagcgc ttggtttaat 8460
   gacggcttgt ttcttttctg tggctgcgtg aaagccttga ggggctccgg gagggccctt 8520
   tgtgcggggg gagcggctcg gggctgtccg cggggggacg gctgccttcg ggggggacgg 8580
   ggcagggcgg ggttcggctt ctggcgtgtg accggcggct ctagagcctc tgctaaccat 8640
   gttcatgcct tcttcttttt cctacagctc ctgggcaacg tgctggttat tgtgctgtct 8700
   catcattttg gcaaagaatt ggatcggacc gaaattaata cgactcacta ta 8752

<210> 12
<211> 6490
<212> DNA
<213> artificial

<220>
<223> cDNA kodierend für die ATP4B-Untereinheit ligiert in den Vector
      pTriEx

<400> 12
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatgg cggctctgca 180

ggagaagaag acgtgtggcc agcgcatgga ggagttccag cgttactgct ggaacccgga 240

cacggggcag atgctgggcc gcaccctgtc ccggtgggtg tggatcagcc tgtactacgt 300

ggccttctac gtggtgatga ctgggctctt cgccctgtgc ctctatgtgc tgatgcagac 360

agtggacccg tacacaccgg actaccaaga ccagctacgg tcaccagggg taaccttaag 420

gccggatgtt tacggggaga aaggcctgga aattgtctac aacgtctctg ataacagaac 480

ctgggcagac ctcacacaga ctctccacgc cttcctagca ggctactctc cagcagccca 540

ggaggacagc atcaactgca cctccgagca gtacttcttc caggagagtt tccgcgctcc 600

caaccacacc aagttctcct gcaagttcac ggcagatatg ctgcagaact gctcaggcct 660

ggcggatccc aacttcggct ttgaagaagg aaagccatgt tttattatta aaatgaacag 720

gatcgtcaag ttcctcccca gcaacggctc ggcccccaga gtggactgcg ccttcctgga 780

ccagccccgc gagctcggcc agccgctgca ggtcaagtac taccctccca acggcacctt 840

cagtctgcac tacttccctt attacgggaa gaaagcccag ccccactaca gcaaccccct 900

ggtggcagcg aagctcctca acatccccag gaacgctgag gtcgccatcg tgtgcaaggt 960

catggcagag cacgtgacct tcaacaatcc ccacgacccg tatgaaggga aagtggagtt 1020

caaactcaag attgagaagt gactcgagca ccaccatcac catcaccatc actaagtgat 1080

taacctcagg tgcaggctgc ctatcagaag gtggtggctg gtgtggccaa tgccctggct 1140

cacaaatacc actgagatcg atctttttcc ctctgccaaa aattatgggg acatcatgaa 1200

gccccttgag catctgactt ctggctaata aaggaaattt attttcattg caatagtgtg 1260

ttggaatttt ttgtgtctct cactcggaag gacatatggg agggcaaatc atttaaaaca 1320

tcagaatgag tatttggttt agagtttggc aacatatgcc catatgtaac tagcataacc 1380

ccttggggcc tctaaacggg tcttgagggg ttttttgctg aaagcatgcg gaggaaattc 1440

tccttgaagt ttccctggtg ttcaaagtaa aggagtttgc accagacgca cctctgttca 1500

ctggtccggc gtattaaaac acgatacatt gttattagta catttattaa gcgctagatt 1560

ctgtgcgttg ttgatttaca gacaattgtt gtacgtattt taataattca ttaaatttat 1620

aatctttagg gtggtatgtt agagcgaaaa tcaaatgatt ttcagcgtct ttatatctga 1680

atttaaatat taaatcctca atagatttgt aaaataggtt tcgattagtt tcaaacaagg 1740

gttgtttttc cgaaccgatg gctggactat ctaatggatt ttcgctcaac gccacaaaac 1800

ttgccaaatc ttgtagcagc aatctagctt tgtcgatatt cgtttgtgtt ttgttttgta 1860

ataaaggttc gacgtcgttc aaaatattat gcgcttttgt atttctttca tcactgtcgt 1920

tagtgtacaa ttgactcgac gtaaacacgt taaatagagc ttggacatat ttaacatcgg 1980

gcgtgttagc tttattaggc cgattatcgt cgtcgtccca accctcgtcg ttagaagttg 2040

cttccgaaga cgattttgcc atagccacac gacgcctatt aattgtgtcg gctaacacgt 2100

ccgcgatcaa atttgtagtt gagctttttg gaattatttc tgattgcggg cgtttttggg 2160

cgggtttcaa tctaactgtg cccgatttta attcagacaa cacgttagaa agcgatggtg 2220

caggcggtgg taacatttca gacggcaaat ctactaatgg cggcggtggt ggagctgatg 2280

ataaatctac catcggtgga ggcgcaggcg gggctggcgg cggaggcgga ggcggaggtg 2340

gtggcggtga tgcagacggc ggtttaggct caaatgtctc tttaggcaac acagtcggca 2400

cctcaactat tgtactggtt tcgggcgccg tttttggttt gaccggtctg agacgagtgc 2460

gatttttttc gtttctaata gcttccaaca attgttgtct gtcgtctaaa ggtgcagcgg 2520

gttgaggttc cgtcggcatt ggtggagcgg gcggcaattc agacatcgat ggtggtggtg 2580

gtggtggagg cgctggaatg ttaggcacgg gagaaggtgg tggcggcggt gccgccggta 2640

taatttgttc tggtttagtt tgttcgcgca cgattgtggg caccggcgca ggcgccgctg 2700

gctgcacaac ggaaggtcgt ctgcttcgag gcagcgcttg gggtggtggc aattcaatat 2760

tataattgga atacaaatcg taaaaatctg ctataagcat tgtaatttcg ctatcgttta 2820

ccgtgccgat atttaacaac cgctcaatgt aagcaattgt attgtaaaga gattgtctca 2880

agctcggaac gctgcgctcg gtcgttcggc tgcggcgagc ggtatcagct cactcaaagg 2940

cggtaatacg gttatccaca gaatcagggg ataacgcagg aaagaacatg tgagcaaaag 3000

gccagcaaaa ggccaggaac cgtaaaaagg ccgcgttgct ggcgtttttc cataggctcc 3060

gcccccctga cgagcatcac aaaaatcgac gctcaagtca gaggtggcga aacccgacag 3120

gactataaag ataccaggcg tttccccctg gaagctccct cgtgcgctct cctgttccga 3180

ccctgccgct taccggatac ctgtccgcct ttctcccttc gggaagcgtg gcgctttctc 3240

aatgctcacg ctgtaggtat ctcagttcgg tgtaggtcgt tcgctccaag ctgggctgtg 3300

tgcacgaacc ccccgttcag cccgaccgct gcgccttatc cggtaactat cgtcttgagt 3360

ccaacccggt aagacacgac ttatcgccac tggcagcagc cactggtaac aggattagca 3420

gagcgaggta tgtaggcggt gctacagagt tcttgaagtg gtggcctaac tacggctaca 3480

ctagaaggac agtatttggt atctgcgctc tgctgaagcc agttaccttc ggaaaaagag 3540

ttggtagctc ttgatccggc aaacaaacca ccgctggtag cggtggtttt tttgtttgca 3600

agcagcagat tacgcgcaga aaaaaaggat ctcaagaaga tcctttgtta ccaatgctta 3660

atcagtgagg cacctatctc agcgatctgt ctatttcgtt catccatagt tgcctgactc 3720

cccgtcgtgt agataactac gatacgggag ggcttaccat ctggccccag tgctgcaatg 3780

ataccgcgag acccacgctc accggctcca gatttatcag caataaacca gccagccgga 3840

agggccgagc gcagaagtgg tcctgcaact ttatccgcct ccatccagtc tattaattgt 3900

tgccgggaag ctagagtaag tagttcgcca gttaatagtt tgcgcaacgt tgttgccatt 3960

gctacaggca tcgtggtgtc acgctcgtcg tttggtatgg cttcattcag ctccggttcc 4020

caacgatcaa ggcgagttac atgatccccc atgttgtgca aaaaagcggt tagctccttc 4080

ggtcctccga tcgttgtcag aagtaagttg gccgcagtgt tatcactcat ggttatggca 4140

gcactgcata attctcttac tgtcatgcca tccgtaagat gcttttctgt gactggtgag 4200

tactcaacca agtcattctg agaatagtgt atgcggcgac cgagttgctc ttgcccggcg 4260

tcaatacggg ataataccgc gccacatagc agaactttaa aagtgctcat cattggaaaa 4320

cgttcttcgg ggcgaaaact ctcaaggatc ttaccgctgt tgagatccag ttcgatgtaa 4380

cccactcgtg cacccaactg atcttcagca tcttttactt tcaccagcgt ttctgggtga 4440

gcaaaaacag gaaggcaaaa tgccgcaaaa aagggaataa gggcgacacg gaaatgttga 4500

atactcatac tcttcctttt tcaatattat tgaagcattt atcagggtta ttgtctcatg 4560

tccgcgcgtt tcctgcatct tttaatcaaa tcccaagatg tgtataaacc accaaactgc 4620

caaaaaatga aaactgtcga caagctctgt ccgtttgctg gcaactgcaa gggtctcaat 4680

cctatttgta attattgaat aataaaacaa ttataaatgt caaatttgtt ttttattaac 4740

gatacaaacc aaacgcaaca agaacatttg tagtattatc tataattgaa aacgcgtagt 4800

tataatcgct gaggtaatat ttaaaatcat tttcaaatga ttcacagtta atttgcgaca 4860

atataatttt attttcacat aaactagacg ccttgtcgtc ttcttcttcg tattccttct 4920

ctttttcatt tttctcttca taaaaattaa catagttatt atcgtatcca tatatgtatc 4980

tatcgtatag agtaaatttt ttgttgtcat aaatatatat gtctttttta atggggtgta 5040

tagtaccgct gcgcatagtt tttctgtaat ttacaacagt gctattttct ggtagttctt 5100

cggagtgtgt tgctttaatt attaaattta tataatcaat gaatttggga tcgtcggttt 5160

tgtacaatat gttgccggca tagtacgcag cttcttctag ttcaattaca ccatttttta 5220

gcagcaccgg attaacataa ctttccaaaa tgttgtacga accgttaaac aaaaacagtt 5280

cacctccctt ttctatacta ttgtctgcga gcagttgttt gttgttaaaa ataacagcca 5340

ttgtaatgag acgcacaaac taatatcaca aactggaaat gtctatcaat atatagttgc 5400

tctagttatt aatagtaatc aattacgggg tcattagttc atagcccata tatggagttc 5460

cgcgttacat aacttacggt aaatggcccg cctggctgac cgcccaacga cccccgccca 5520

ttgacgtcaa taatgacgta tgttcccata gtaacgccaa tagggacttt ccattgacgt 5580

caatgggtgg actatttacg gtaaactgcc cacttggcag tacatcaagt gtatcatatg 5640

ccaagtacgc cccctattga cgtcaatgac ggtaaatggc ccgcctggca ttatgcccag 5700

tacatgacct tatgggactt tcctacttgg cagtacatct acgtattagt catcgctatt 5760

accatgcatg gtcgaggtga gccccacgtt ctgcttcact ctccccatct cccccccctc 5820

cccaccccca attttgtatt tatttatttt ttaattattt tgtgcagcga tgggggcggg 5880

gggggggggg gggcgcgcgc caggcggggc ggggcggggc gaggggcggg gcggggcgag 5940

gcggagaggt gcggcggcag ccaatcagag cggcgcgctc cgaaagtttc cttttatggc 6000

gaggcggcgg cggcggcggc cctataaaaa gcgaagcgcg cggcgggcgg gagtcgctgc 6060

gacgctgcct tcgccccgtg ccccgctccg ccgccgcctc gcgccgcccg ccccggctct 6120

gactgaccgc gttactccca caggtgagcg ggcgggacgg cccttctcct tcgggctgta 6180

attagcgctt ggtttaatga cggcttgttt cttttctgtg gctgcgtgaa agccttgagg 6240

ggctccggga gggccctttg tgcgggggga gcggctcggg gctgtccgcg gggggacggc 6300

tgccttcggg ggggacgggg caggcggggg ttcggcttct ggcgtgtgac cggcggctct 6360

agagcctctg ctaaccatgt tcatgccttc ttctttttcc tacagctcct gggcaacgtg 6420

ctggttattg tgctgtctca tcattttggc aaagaattgg atcggaccga aattaatacg 6480

actcactata 6490